# EUROPÄISCHE PATENTSCHRIFT

(11) **EP 2 379 743 B1**
(45) Veröffentlichungstag und Bekanntmachung des Hinweises auf die Patenterteilung: **03.05.2017**
(21) Anmeldenummer: 09798892.7
(22) Anmeldetag: 21.12.2009
(51) Int. Cl.: C12Q 1/68

(54) **VERFAHREN ZUR BESTIMMUNG DER PRÄDISPOSITION FÜR MORBUS CROHN**
METHOD FOR DETERMINING PREDISPOSITION FOR CROHNS DISEASE
PROCÉDÉ POUR DETERMINER UNE PRÉDISPOSITION POUR LA MALADIE DE CROHN

(30) Priorität: 22.12.2008 DE 102008064509
(43) Veröffentlichungstag der Anmeldung: 26.10.2011
(73) Patentinhaber: Robert Bosch Gesellschaft für medizinische Forschung mbH, 70376 Stuttgart (DE)
(72) Erfinder: WEHKAMP, Jan, 70372 Stuttgart (DE); STANGE, Eduard, 70469 Stuttgart (DE); KOSLOWSKI, Maureen, 70195 Stuttgart (DE)
(74) Vertreter: Bee, Joachim
(86) Internationale Anmeldenummer: PCT/EP2009/009176
(87) Internationale Veröffentlichungsnummer: WO 2010/072389

(56) Entgegenhaltungen:
- WO-A1-2006/116867
- US-A1- 2006 263 791
- WEHKAMP J ET AL: "NOD2 (CARD15) mutations in Crohn's disease are associated with diminished mucosal alpha-defensin expression." GUT NOV 2004, Bd. 53, Nr. 11, November 2004 (2004-11), Seiten 1658-1664, XP002569876 ISSN: 0017-5749
- WEHKAMP JAN ET AL: "Reduced Paneth cell alpha-defensins in ileal Crohn's disease." PROCEEDINGS OF THE NATIONAL ACADEMY OF SCIENCES OF THE UNITED STATES OF AMERICA 13 DEC 2005, Bd. 102, Nr. 50, 13. Dezember 2005 (2005-12-13), Seiten 18129-18134, XP002569877 ISSN: 0027-8424
- GASCHE C ET AL: "Genotypes and phenotypes in Crohn's disease: do they help in clinical management?" GUT JAN 2005, Bd. 54, Nr. 1, Januar 2005 (2005-01), Seiten 162-167, XP002569878 ISSN: 0017-5749
- WEHKAMP JAN ET AL: "The Paneth cell alpha-defensin deficiency of ileal Crohn's disease is linked to Wnt/Tcf-4." JOURNAL OF IMMUNOLOGY (BALTIMORE, MD. : 1950) 1 SEP 2007, Bd. 179, Nr. 5, 1. September 2007 (2007-09-01), Seiten 3109-3118, XP002569879 ISSN: 0022-1767
- DATABASE SNP [Online] 12. August 2002 (2002-08-12), XP002569880 gefunden im NCBI Database accession no. rs3814570
- DATABASE SNP [Online] 17. November 2003 (2003-11-17), XP002569881 gefunden im NCBI Database accession no. rs10885394
- DATABASE SNP 17. November 2003 (2003-11-17), XP002569882 gefunden im NCBI Database accession no. rs10885395
- DUVAL A ET AL: "Frequent frameshift mutations of the TCF-4 gene in colorectal cancers with microsatellite instability." CANCER RESEARCH 1 SEP 1999, Bd. 59, Nr. 17, 1. September 1999 (1999-09-01), Seiten 4213-4215, XP002569883 ISSN: 0008-5472
- JIANG YING ET AL: "Association of hTcf-4 gene expression and mutation with clinicopathological characteristics of hepatocellular carcinoma." WORLD JOURNAL OF GASTROENTEROLOGY : WJG OCT 2002, Bd. 8, Nr. 5, Oktober 2002 (2002-10), Seiten 804-807, XP002569884 ISSN: 1007-9327
- KOSLOWSKI MAUREEN J ET AL: "Selective influence of Tcf-4 mediated wnt signaling on intestinal innate and adaptive immunity of ileal Crohn's disease" GASTROENTEROLOGY; DIGESTIVE DISEASE WEEK MEETING/109TH ANNUAL MEETING OF THE AMERICAN-GASTROENTEROLOGICAL-ASSOCIATION; SAN DIEGO, CA, USA; MAY 17 -22, 2008, ELSEVIER, PHILADELPHIA, PA, Bd. 134, Nr. 4, Suppl. 1, 1. April 2008 (2008-04-01), Seite A514, XP008119394 ISSN: 0016-5085 [gefunden am 2008-07-31]

## Beschreibung

Die vorliegende Erfindung betrifft ein Verfahren zur Bestimmung einer Prädisposition eines Lebewesens für Morbus Crohn, bei welchem aus einer biologischen Probe des Lebewesens das Vorliegen von Einzelnucleotid-Polymorphismen (SNP) bestimmt wird. Ferner betrifft die Erfindung Primer und allelspezifische Sonden zum Nachweis des Vorliegens oder Nichtvorliegens eines SNPs, diagnostische Kits, welche mindestens einen solchen Primer oder eine solche allelspezifische Sonde aufweisen, sowie die Verwendung bestimmter SNPs zur Bestimmung einer Prädisposition eines Lebewesens für Morbus Crohn.

Chronisch entzündliche Darmerkrankungen umfassen zwei voneinander verschiedene Krankheitsbilder, Colitis ulcerosa und Morbus Crohn. Beide Erkrankungen führen durch starke Entzündungsreaktionen des Darmes, welche mit starken Durchfällen, Stuhlinkontinenz und weiteren Beschwerden einhergehen, zu einer nachhaltigen Beeinträchtigung der Lebensqualität. Darüber hinaus werden beide Erkrankungen mit einer Erhöhung des Darmkrebsrisikos in Verbindung gebracht.

Beide Erkrankungen treten in den westlichen Industriestaaten in einer hohen Frequenz von 150 Erkrankten pro 100.000 Personen (Morbus Crohn) und 80 Erkrankten pro 100.000 Personen (Colitis ulcerosa) auf. In den Industrie- wie auch den Entwicklungsländern steigt darüber hinaus seit Jahrzehnten die Tendenz des Auftretens chronisch entzündlicher Darmerkrankungen.

Die genannten chronisch entzündlichen Darmerkrankungen gelten hierbei als multifaktoriell verursacht, wobei genetische Prädispositionen, verschiedene Umweltfaktoren sowie veränderte Immunreaktionen von Bedeutung sind, die zur Zerstörung der mukosalen Barriere führen und durch die Darmflora ausgelöst bzw. unterhalten werden.

So werden beispielsweise Mutationen in MDR1-Gen, welches für das P-Glycoprotein 170 codiert, mit einem erhöhten Risiko für ein Erkranken an ulcerativer Colitis in Verbindung gebracht. Auch ist eine Reihe von genetischen Faktoren bekannt, welche das Auftreten von Morbus Crohn begünstigen könnten, beispielsweise Mutationen in den Genen von NOD2, sowie OCTN1 und 2; (siehe Suchy et al.: "Inflammatory response gene polymorphisms and their relationship with colorectal cancer risk", BMC Cancer 2008;8:112; Okazaki et al.: "Contributions of IBD5, IL23R, ATG16L1, and NOD2 to Crohn's disease risk in a population-based case-control study: Evidence of gene-gene interactions", Inflamm. Bowel Dis. 2008;14:1528-1541; Henckaerts L. et al.: "The role of genetics in inflammatory bowel disease", Curr. Drug Targets 2008;9(5):361-8).

Insgesamt ergibt sich hieraus ein großer Bedarf an diagnostischen Mitteln, um einerseits mittels genetischer Diagnostik das Risiko eines Patienten, an einer chronisch entzündlichen Darmerkrankung zu erkranken, zu bewerten, so dass dem Patienten Verhaltensanpassungen empfohlen werden können, um das Erkrankungsrisiko zu verringern. Andererseits soll es auch möglich sein, mittels genetischer Diagnostik die molekularen Ursachen einer individuellen chronisch entzündlichen Darmerkrankung zu diagnostizieren und eine daran angepasste Therapie einzuleiten.

Im Stand der Technik sind mehrere Verfahren zur genetischen Diagnostik entzündlicher Darmerkrankungen bekannt.

So beschreibt die WO 2004/083232 A2 ein Verfahren zur Diagnostik der genetischen Prädisposition eines Individuums für eine chronisch entzündliche Darmerkrankung, in welchem SNPs im CCRL2-Gen nachgewiesen werden können. Solche Mutationen zeigen eine Korrelation mit dem Auftreten von Colitis ulcerosa.

Die US 2006/0141478 A1 beschreibt eine diagnostische Methode, in der Promotervarianten des NF-κB-Gens diagnostiziert und mit dem Auftreten chronisch entzündlicher Darmerkrankungen, sowohl Morbus Crohn als auch Colitis ulcerosa korreliert werden.

Ferner offenbaren Wehkamp et al. ("The Paneth cell alpha-defensin deficiency of ileal Crohn's disease is linked to Wnt/Tcf-4", J. Immunol. 179(5): 3109-3118 (2007)) einen Zusammenhang zwischen einem alpha-Defensin-Mangel und dem Transkriptionsfaktor TCF4 bei Morbus Crohn. Dabei wurde festgestellt, dass bei einer reduzierten TCF4-Expression auch eine reduzierte alpha-Defensin-Produktion zu beobachten ist.

Die im Stand der Technik bekannten Diagnoseverfahren weisen jedoch eine durchweg beschränkte Aussagekraft auf. So können Risiken, an einer chronisch entzündlichen Darmerkrankung zu erkranken, im Vorfeld nur unzureichend erkannt werden. Auch ist es, beispielsweise durch die Diagnose von Promotervarianten des NF-κB-Gens, nicht möglich, zwischen verschiedenen Varianten chronisch entzündlicher Darmerkrankungen zu differenzieren.

Vor diesem Hintergrund liegt der Erfindung die Aufgabe zugrunde, bestehende Diagnoseverfahren zu verbessern, indem die Vorhersagegenauigkeit erhöht und die Differenzierung verschiedener chronisch entzündlicher Darmerkrankungen erleichtert wird.

Diese Aufgabe wird erfindungsgemäß durch ein Verfahren zur Bestimmung einer Prädisposition eines Lebewesens für Morbus Crohn gelöst, bei dem in einer biologischen Probe des Lebewesens das Vorliegen von SNPs bestimmt wird, welches dadurch gekennzeichnet ist, dass das zumindest eine SNP in mindestens einem Gen bestimmt wird, das für ein mit dem Wnt-Signalweg in Paneth-Zellen assoziiertes Protein codiert, wobei das zumindest eine Gen TCF4 oder LRP6 ist, und wobei das zumindest eine SNP ausgewählt ist aus einem der folgenden: rs3814570, rs10885394, rs10885395 (TCF 4) und rs2302685 (LRP6).

Bei einem Vorliegen des zumindest einen SNP wird die Prädisposition des Lebewesens zur Entwicklung von Morbus Crohn bestimmt.

Die Aufgabe wird somit durch die vorliegende Erfindung vollständig gelöst.

Mit "SNPs" (engl. Single Nucleotide Polymorphisms) oder "Einzelnucleotidpolymorphismen", wie die Begriffe hierin synonym verwendet werden, werden Variationen von einzelnen Basenpaaren in einem DNA-Strang im Vergleich zum Wildtyp in einer bestimmten Population bezeichnet. SNPs stellen ca. 90 % aller genetischen Varianten im menschlichen Genom dar, und treten ungleichmäßig stark an bestimmten Regionen im Genom auf. Sie stellen Mutationen dar, d. h. genetische Veränderungen, die sich zu einem gewissen Grad im Genpool einer Population durchgesetzt haben. Dabei können die SNPs als Substitutionen auftreten, bei welchen eine Base, bspw. Cytosin, gegen eine andere Base, bspw. Thymin, ausgetauscht ist, oder aber als Deletionen oder Insertionen.

SNPs weisen dabei immer einen von zwei oder sehr selten auch mehrerer Zuständen auf und werden allelisch vererbt. Die Mehrzahl der bekannten SNPs betrifft nicht codierende Bereiche im Genom, d.h. Regionen, die entweder zwischen Genen oder zwischen Exonbereichen einzelner Gene liegen. Grundsätzlich können diese Genvarianten in nicht- codierenden Bereichen auch regulatorische Sequenzen, z.B. Promotoren, Enhancer oder Spleißstellen betreffen und damit Auswirkungen auf die Expression von Genen haben. SNPs die direkt die kodierende Sequenz betreffen können still sein, d.h. der Basenaustausch verändert nicht die Übersetzung des entsprechenden Triplettcodes in die analoge Aminosäure und hat somit also keinen Einfluss auf die Peptidsequenz. Allerdings können sich aus unterschiedlicher Häufigkeit äquivalenter t-RNAs für spezifischen Basentripletts Unterschiede für die Effizienz der Translation ergeben, sowie stille SNPs zu verändeter Sekundärstruktur der mRNA(z.B. "hairpin") führen und somit kann die Expression bestimmter Gene post-transkriptionell durch stille SNPs beeinflusst werden. Einige SNPs haben codierende Funktion, d.h. die unterschiedlichen Allele führen zu einem unterschiedlichen Einbau einer Aminosäure in das entstehende Peptid ("Missense SNP"), mit der Folge dass dessen Funktion verändert werden kann. Wenn ein SNP die Aminosäuresequenz eines Proteins nicht verändert, dann ist der SNP gewöhnlich "stumm".

Der Ausdruck "Allel" bezieht sich vorliegend - wie auch im entsprechenden Fachgebiet - auf alternative Formen von Genen oder Abschnitten davon. Allele besetzen auf homologen Chromosomen den gleichen Locus; dies bedeutet, dass dann, wenn ein Subjekt zwei identische Allele eines Gens besitzt, dieses Subjekt homozygot für dieses Allel ist, und wenn das Subjekt unterschiedliche Allele eines spezifischen Gens besitzt, dieses Subjekt heterozygot für das Allel ist. Dabei können sich Allele eines spezifischen Gens voneinander in einem einzelnen Nucleotid unterscheiden; ferner kann ein Allel eines Gens auch in Form eines Gens mit einer oder mehreren Mutationen oder Sequenz-Varianten vorliegen. Bei dem erfindungsgemäßen Verfahren kann jeweils ein Allel oder beide Allele auf das Vorliegen des SNP untersucht werden.

Dabei wird unter einem Nucleotid einer Nucleinsäure, die als DNA oder RNA vorliegen kann, Adenin, Cytosin, Guanin, Thymin verstanden - im Falle von RNA Uracil statt Thymin.

Im Rahmen der vorliegenden Erfindung wird unter "SNP" insbesondere eine Punktmutation im Genom eines Lebewesens verstanden, durch die es zum Austausch der, gemäß der in der jeweiligen Spezies vorherrschenden genetischen Norm, an einer bestimmten Position vorliegenden Base eine bestimmte andere Base kommt. Dabei wird üblicherweise, wenn bei einem SNP die gleiche Position betroffen ist, stets die gleiche "rs-Nummer" vergeben; der Polymorphismus wird dann entsprechend dem Beispiel mit C/A/ oder G angegeben.

Im Genom können SNPs, wenn sie bi-allelisch vorliegen, in drei möglichen Genotypen vorkommen, nämlich in einer von zwei möglichen homozygoten Formen (Allel 1/ Allel 1 oder Allel 2 / Allel 2) oder aber in einer heterozygoten Form (Allel 1/ Allel 2). Benachbarte SNPs können in unterschiedlichem Ausmaß miteinander gelinkt sein, d.h., zu einem gewissen Prozentsatz treten sie in der Bevölkerung in einer bestimmten Kombination nur gemeinsam auf und bilden damit einen sogenannten Haplotyp.

Unter "Lebewesen" sollen vorliegend insbesondere Säugetiere, insbesondere der Mensch, verstanden werden.

Unter "biologische Probe" wird vorliegend jede Probe verstanden, die genetisches Material enthält, und die einem Patienten, insbesondere einem Menschen, entnommen wurde, und die dann *in vitro* für die Zwecke des erfindungsgemäßen Diagnoseverfahrens eingesetzt wird. Die biologische Probe umfasst dabei sowohl Proben aus Körperflüssigkeiten, die Zellen enthalten, wie bspw. Blut, Speichel, etc., oder aus Gewebe, inkl. Organen.

Unter "Linkage" bzw. "Kopplung" wird vorliegend die Tendenz der Gene, Allele, Loci oder genetischen Marker beschrieben, als Ergebnis ihrer Lokalisierung auf dem gleichen Chromosom gemeinsam vererbt zu werden. Der Ausdruck "Linkage Disequilibrium" bzw. "Kopplungs-Ungleichgewicht" (nachstehend auf mit "LD" bezeichnet) bezieht sich auf eine mehr als zufällige Assoziierung zwischen spezifischen Allelen an zwei Markerloci innerhalb einer bestimmten Population. Allgemein sinkt as Kopplungs-Ungleichgewicht bei einem Anstieg der physikalischen Entfernung der Loci.

Der Ausdruck "bestimmen", wie er vorliegend für die Bestimmung der SNPs benutzt wird, bezieht verschiedene Methoden und Verfahren zur Analyse eines oder mehrerer SNP an einer bestimmten Stelle im Genom, wobei der Ausdruck sowohl die direkte Bestimmung, d.h. bspw. die Sequenzierung, als auch die indirekte Bestimmung, also bspw. die Amplifizierung und/oder Hybridisierung, mit einschließt.

Vorliegend wird die Erkrankung Morbus Crohn auch mit CD abgekürzt, herrührend aus dem Englischen (Crohn's Diesease), sowie die chronisch entzündliche Darmerkrankung mit IBD, eine ebenfalls aus dem Englischen herrührende Abkürzung (Inflammatory Bowel Disease).

Die Erfinder haben nun erkannt, dass es überraschenderweise möglich ist, anhand bestimmter SNPs in den genomischen Regionen von Faktoren des WNT-Signalwegs in Paneth-Zellen eine genetische Prädisposition für Morbus Crohn, spezifischer Morbus Crohn des Dünndarms, zu diagnostizieren.

Die intestinale Barrierefunktion ist ein zentraler Faktor in der Pathogenese chronisch entzündlicher Darmerkrankungen, wie Morbus Crohn. Im menschlichen Darm bilden die Epithelzellen zum einen eine physische Schranke, zum anderen produzieren sie als Teil der angeborenen Immunabwehr eine wirkungsvolle chemische sowie biologische Schutzschicht um das Eindringen von Mikroben zu verhindern. Im Dünndarm finden sich zum vergleichsweise dicht besiedelten Dickdarm relativ wenige Bakterien, trotz allem erreicht ihre Anzahl im Lumen 10⁵- 10⁷ pro ml Flüssigkeit. Trotz dieser ständigen Anwesenheit von Mikroorganismen, kommt es im gesunden Menschen nur selten zu Infektionen und Entzündungsreaktionen. Eine wichtige Rolle in diesem Zusammenhang spielen die antimikrobiellen Produkte der Panethzellen. Diese Zellen kommen spezifisch in der Basis der Krypten des Dünndarms vor, was sie von den anderen ausdifferenzierten Zelltypen des intestinalen Epithels unterscheidet. Sie gehen von multipotenten adulten Stammzellen aus, die zwischen und/oder über den Panethzellen liegen und haben *per definitionem* die Fähigkeit zur Selbsterneuerung. Von ihnen abstammende Tochterzellen bilden eine Zone von Vorläuferzellen (transient amplifying cells), die nach 4-6 Zellteilungen in die verschiedenen post- mitotischen Zelltypen, die Absorptiven und Enteroendokrinen Zellen und die Becher- sowie Panethzellen des Epithels differenzieren.

Eine wichtige Funktion im Erhalt der intestinalen Stammzellen sowie in der Differenzierung von Panethzellen nimmt der Wnt- Signalweg ein. In adulten Panethzellen ist dessen Aktivität außerdem für die korrekte Funktion der Zelle unabdingbar, da er direkt die Expression antimikrobieller Substanzen, der Panethzell α- Defensine HD-5 (DEFA5) und -6 (DEFA6) vermittelt.

Bei Patienten mit Morbus Crohn des Dünndarms ist die Expression dieser Substanzen verringert und kann somit zu einer Einschränkung der intestinalen Immunabwehr in diesem Bereich führen. Eine Schwächung der Schutzbarriere kann Bakterien das Eindringen in das Epithel ermöglichen und somit zu der Entstehung einer adaptiven Immunreaktion sowie einer Entzündung führen. Die spezifische Reduktion von HD-5 und -6 bei Morbus Crohn des Dünndarm, lässt auf eine wichtige Rolle der Panethzelle bei der Entstehung dieser Erkrankung schließen, welche auch durch mehrere genetische Studien untermauert wird.

Es ist zwar beispielsweise bekannt, dass Morbus-Crohn-Patienten mit einem bestimmten Polymorphismus T300A des *ATG16 autophagy related 16-like 1* (*ATG16L1*) Gens eine gestörte Exozytose der Granula von Panethzellen aufweisen (Cadwell et al., "A key role for autophagy and the autophagy gene Atg1611 in mouse and human intestinal Paneth cells", Nature 2008; 456(7219):259.63). Die Granula von Panethzellen stellen eine Art Vorratskammer für antimikrobielle Substanzen dar und ihre Unversehrtheit ist essentiell für die Sezernierung dieser Wirkstoffe.

Ein weiteres Gen, das stark mit Morbus Crohn des Dünndarms assoziiert ist, ist das bakterielle Erkennungsmolekül "Nucleotide-binding oligomerization domain containing 2" (NOD2), auch bekannt als CARD15. Dieser Muramyldipeptid- Rezeptor liegt intrazellulär in Panethzellen vor, und eine framshift-Mutation, die zu einer verkürzten Form des Proteins führt, steht mit einer besonders niedrigen Expression von Panethzell α- Defensinen in Zusammenhang (Wehkamp et al., "NOD2 (CARD15) mutations in Crohn's disease are associated with diminished mucosal α-defensin expression", Gut 2004;53:1658-64; Wehkamp et al.: "Reduced Paneth (alpha)-defensins in ileal Crohn's Diesease", PNAS 2005;102:18129-34).

Wnt-Proteine sind eine Familie sekretierter Morphogene, die eine wichtige Rolle bei der Regulierung der Zelldifferenzierung im adulten Organismus sowie der Differenzierung während der Embryogenese spielen. Der Wnt-Signalweg wird durch das Binden eines Proteins aus der Wnt-Familie an Frizzled u.a. Zelloberflächenrezeptoren induziert. Diese Induktion wird durch sogenannte LRP-Proteine ("low density lipoprotein receptor-related Protein"), welche ebenfalls als Rezeptoren wirken, begünstigt. Durch die Bindung des Wnt-Proteins an Oberflächenrezeptoren werden intrazellulär verschiedene Signaltransduktionspfade, beispielsweise über das Protein Dishevelled, aktiviert. So inhibiert bzw. inaktiviert Dishevelled einen Glycogen Synthase Kinase 3 (GSK3) enthaltenden Proteinkomplex, der normalerweise β-Catenin dem Abbau durch das Proteasom aussetzt und somit dessen Akkumulation verhindert. Hierdurch wird ein zytoplasmatischer Pool des Proteins β-Catenin stabilisiert, kann in den Zellkern gelangen, und dort im Zusammenwirken mit Transkriptionsfaktoren der Lef/TCF-Familie eine Reihe von Zielgenen aktivieren.

Zwar war bekannt, dass die Differenzierung der Paneth-Körnerzellen sowie die Sekretion der humanen Alpha-Defensine (HD) 5 und 6 durch Proteine aus der Familie der TCF-Transkriptionsfaktoren zumindest mitbestimmt wird, und dass bei einigen an Morbus Crohn erkrankten Personen eine verringerte Expression von Defensinen einerseits und Tcf4 andererseits festzustellen ist, es war jedoch nicht zu erwarten, dass bestimmte, mit den Genen des Wnt-Signalweges assoziierte, genetische Variationen eine deutliche Assoziation mit dem Auftreten von Morbus Crohn, insbesondere Morbus Crohn im Dünndarm aufweisen würden.

Die Erfinder der vorliegenden Anmeldung haben nun zum ersten Mal erkannt, dass Gene, die wichtige Funktionen im Wnt Signalweg einnehmen, Veränderungen im Zusammenhang mit der Erkrankung zeigen. In eigenen Versuchen konnten die Erfinder zeigen, dass Mutationen in wichtigen Genen von am Wnt-Signalweg beteiligten Faktoren/Proteine diesen und damit die Entwicklung und die Funktion der Paneth-Zellen beeinflussen.

Hierbei hat das neue Verfahren den Vorteil, dass, vorzugsweise in Kombination mit der Diagnose des Vorliegens oder Nichtvorliegens bekannter krankheitsassoziierter SNPs, beispielsweise in den Genen von NOD2, ATG16L1, DLG5, die Vorhersagegenauigkeit für das Auftreten oder Nichtauftreten von Morbus Crohn erhöht wird.

Das nunmehr von den Erfindern bereitgestellte Diagnoseverfahren mit der Bestimmung von SNP(s) im Wnt-Signalweg ermöglicht daher auch eine erleichterte Differentialdiagnostik, da mit dem erfindungsgemäßen Verfahren dann bspw. Morbus Crohn gegenüber bakteriellen Infektionen wie Tuberkulose oder Yersiniose effizienter differenziert werden kann. Dies ermöglicht vorteilhafterweise einerseits eine schnelle positive Diagnose und andererseits auch einen schnellen, spezifische Therapieansatz.

Daher betrifft die vorliegende Erfindung auch ein Verfahren zur differentiellen Diagnose entzündlicher Darmerkrankungen zur Unterscheidung von Morbus Crohn und den jeweils anderen entzündlichen bzw. infektiösen Darmerkrankungen. Bei dem erfindungsgemäßen Verfahren wird ebenfalls in einer biologischen Probe des Lebewesens das Vorliegen von zumindest einem Einzelnucleotid-Polymorphismus (SNP) bestimmt, das für ein mit dem Wnt-Signalweg in Paneth-Zellen assoziiertes Protein codiert.

Mit dem neuen Verfahren ist es erstmals möglich, die individuell wahrscheinliche Ausprägung der Erkrankung zu prognostizieren. So kann das neue Verfahren dazu genutzt werden, die Wahrscheinlichkeit eines Auftretens von Morbus Crohn mit oder ohne Beteiligung des Dünndarms differentiell zu prognostizieren.

Weiterhin kann das Verfahren Aufschluss über die individuelle Ursache oder mögliche spätere Ursache von Morbus Crohn geben. Hierbei kann spezifisch ein am Entstehungsweg von Morbus Crohn beteiligter genetischer Defekt identifiziert werden, so dass genau bestimmt werden kann, an welcher Stelle des beteiligten WNT-Signalwegs eine Fehlregulation eingetreten ist oder noch eintreten wird.

Auf Basis einer solchen Diagnose ist es erstmals möglich, Morbus Crohn ursächlich zu therapieren.

Im Gegensatz hierzu ist es mit den durch das neue Verfahren gewonnenen Daten möglich, eine spezifisch auf den individuellen Patienten abgestimmte Therapie, beispielsweise eine Gentherapie, zu konzipieren und durchzuführen. Hierbei könnten durch in den Darm eingebrachte Vektoren, beispielsweise Viren, spezifisch die Zellen bzw. Gewebe infiziert und transformiert werden, welche von Morbus Crohn betroffen sind. Somit könnte ein funktionierender Signalweg erzeugt und der Ausbildung von Morbus Crohn entgegengewirkt werden.

Insbesondere ist bei dem erfindungsgemäßen Verfahren bevorzugt, wenn zumindest ein SNP in zumindest einem der folgenden Gene bestimmt wird: TCF4 (auch als TCF7L2 bezeichnet, HGNC (HUGO Gene Nomenclature Committee)-ID: 11641), oder LRP6 (HGNC:6698). (Zur Identifizierung der Gene siehe unter www.genenames.org).

Unter den angegebenen "Genen" wird vorliegend stets die kodierende Sequenz der angegebenen Gene sowie die Intron-Sequenzen und die 5'- und 3'-untranslatierten/regulatorischen Regionen der genannten Gene verstanden.

Die Erfinder der vorliegenden Anmeldung legen Daten bezüglich Tcf4, dem Wnt Tanskriptionsfaktor, der direkt HD5 und HD6 induziert, sowie bezüglich der Low Density Lipoprotein Receptor-Related Proteine LPR5 und LPR6, wichtigen Rezeptormolekülen des Signalwegs, vor, anhand derer eindeutig ein Zusammenhang zwischen einer Veränderung in Genen, die für mit dem Wnt-Signalweg assoziierte Proteine codieren, und der Erkrankung Morbus Crohn, insbesondere des Dünndarms, gezeigt werden konnte. Die bereitgestellten Daten lassen zeigen damit, dass Faktoren, die die Funktion von Panethzellen mitbestimmen, wichtige Kandidaten in der Pathogenese von Morbus Crohn darstellen.

Ferner betrifft die Erfindung die Verwendung von Primern und allelspezifischen Sonden bzw. Oligonucleotide zum Nachweis des Vorliegens oder Nichtvorliegens eines SNPs, sowie die Verwendung von SNPs in mindestens einem Gen aus der Gruppe TCF4 oder LRP6 zur Bestimmung einer Prädisposition eines Lebewesens für Morbus Crohn. Allgemein kann ein Polymorphismus in der genomischen DNA, in der mRNA oder in der cDNA nachgewiesen werden.

Dabei wird unter "Primer" (oder "Sonde" oder "Oligonucelotidsonde") ein Oligonucleotid bzw. eine einzelsträngige Aufeinanderfolge von Nucleinsäure verstanden, die in Kombination mit DNA-replizierenden Enymen, bspw. der DNA-Polymerase als Startpunkt eingesetzt werden, um bspw. anhand einer sogenannten "Template"-DNA eine bestimmte Region zu amplifizieren (insbesondere bei der Polymerase-Ketten-Reaktion (PCR); mit Hilfe der Primer lässt sich also der spezifische DNA-Abschnitt, der amplifiziert werden soll, festlegen. Sonden sind meist mit einer molekularen (bspw. radioaktiven) Markierung versehen, und werden in molekularbiologischen Hybridisierungsverfahren zur Sequenz-spezifischen Detektion von DNA- und RNA-Molekülen eingesetzt. Ein Sequenz-spezifischer Primer/Sonde hybridisiert nicht mit Allelen eines Genlocus, der den Sequenzpolymorphismus, für den der Primer/die Sonde spezifisch ist, nicht enthält.

Die Primer, die gemäß der vorliegenden Erfindung zur Sequenz-spezifischen Detektion der SNPs geeignet sind, umfassen daher eine Sequenz, die den Polymorphismus vorzugsweise überlappt, bzw. diesen flankiert.

Insbesondere ist also bevorzugt, wenn bei dem erfindungegemäßen Verfahren zumindest ein SNP detektiert wird, der ausgewählt ist aus der Gruppe umfassend die SNPs mit den Referenznummern rs3814570, rs10885394, rs10885395, rs2302685.

Die hierin offenbarten SNPs werden den folgenden Genen zugeordnet:

**Tabelle 1: Zuordnung der SNPs anhand der rs-Nummern zu ihren Genen:**

| **TCF4** | **LRP5** | **LRP6** |
|---|---|---|
| rs3814570 | rs682429 | rs7302808 |
| rs10885394 | rs4988331 | rs7136380 |
| rs10885395 | rs554734 | rs7308022 |
| | rs312778 | rs2417086 |
| | rs2242339 | rs6488506 |
| | | rs7294695 |
| | | rs12320259 |
| | | rs12313200 |
| | | rs2284396 |
| | | rs2302685 |
| | | rs12314349 |
| | | rs11054701 |
| | | rs11609634 |
| | | rs2417085 |
| | | rs10845494 |
| | | rs1181334 |
| | | rs10772542 |
| | | rs10743980 |
| | | rs7304561 |

Vorliegend bedeutet dabei die "rs" Nummer die Referenznummer für den jeweiligen SNP; unter den jeweils angegebenen Nummern sind bspw. unter http://www.ncbi.nlm.nih.gov/ unter "Entrez SNP" die entsprechenden Sequenzen der SNPs sowie weitere Informationen dazu, wie bspw. die Chromosomen-Lokalisierung der Gene und deren SNPs, zu finden.

So bezieht sich bspw. der SNP mit der Nummer rs3814570 auf einen Polymorphismus mit einem polymorphen Austausch bzw. Substitution der Base Cytosin (C) gegen eine andere Base, insbesondere Thymin (T), an Position -2006 relativ zum Transkriptionsstart-Codon im Promotor des Tcf4-Gens.

Der SNP mit der Nummer rs10885394 bezieht sich auf eine Substitution des Adenins durch eine andere Base, insbesondere Thymin, an der Position -1728 relativ zum Transkriptionsstartcodon im Promotor des Tcf4-Gens, der SNP mit der Nummer rs10885395 auf eine Substitution des Cytosins gegen eine andere Base, insbesondere Thymin, an der Position -1248 relativ zum Transkriptionsstartcodon im Promotor des Tcf4-Gens.

Eine Übersicht über die Positionen der SNPs sowie über die ausgetauschten Basen in den offenbarten Genen findet sich in der nachstehenden Tabelle 2:

**Tabelle 2: Position der SNPs in den Genen Tcf4, LRP5 und LRP6**

| **TCF4** | **Basenaustausch** | **Position*** | |
|---|---|---|---|
| rs3814570 | C/T** | -2006 | 5'angenommener Promotor |
| rs10885394 | A/T | -1728 | |
| rs10885395 | C/T** | -1248 | |
| | | | |

| **LRP5** | | **Position*** | |
|---|---|---|---|
| rs682429 | A/G | -864 | 5'angenommener Promotor |
| rs4988331 | C/T | 5150 | |
| rs554734 | G/T | 112335 | |
| rs312778 | C/T | 28149 | |
| Rs2242339 | C/T | 98942 | |
| | | | |

| **LRP6** | | **Position**** | LRP6: entgegen gesetzte Richtung auf dem Strang |
|---|---|---|---|
| rs7302808 | C/T | -563 | 5'angenommener Promotor |
| rs7136380 | C/T | -566 | |
| rs7308022 | A/G** | 13797 | |
| rs2417086 | A/G | 69556 | |
| rs6488506 | A/G | 101286 | |
| rs7294695 | C/G | 96051 | |
| rs12320259 | C/T | 119500 | |
| rs12313200 | A/G | 135079 | |
| rs 2284396 | C/T | 144734 | |
| rs2302685 | C/T | 117771 | "Missense"-Mutation: V1062I |
| rs1231320C | G/A | 134754 | Tag SNP |
| rs12314349 | A/T | 131848 | Tag SNP |
| rs11054701 | C/T | 130513 | Tag SNP |
| rs11609634 | C/T | 109982 | Tag SNP |
| rs2417085 | C/T | 88294 | Tag SNP |
| rs10772542 | C/T | 25774 | Tag SNP |
| rs1181334 | G/T** | 52081 | Tag SNP |
| rs10845494 | C/T | 70770 | Tag SNP |
| rs1074398C | C/T | 6874 | Tag SNP |
| rs7304561 | A/G | -2736 | Tag SNP |

| | | | |
|---|---|---|---|
| * Position relativ zum Transkriptionsstart ** "Ur"-Allel nicht bekannt | | | |

Die Ur-Allele sind fett markiert, sind jedoch nicht immer die am häufigsten auftretenden

Bei einer Ausführungsform des erfindungsgemäßen Verfahrens zu Bestimmung der Prädisposition eines Patienten/Subjekts zur Entwicklung von Morbus Crohn wird eine biologische Probe des Patienten/Subjekts auf die hierin aufgeführten SNPs untersucht, bzw. das Vorhandensein der verschiedenen SNP- Allele wird detektiert, und die so gewonnene genetische Information wird mit entsprechenden Kontrolldaten und/oder Datenbanken verglichen, die Informationen bezüglich Polymorphismen in den hierin aufgeführten Genen enthalten; basierend auf diesem Vergleich wird die Wahrscheinlichkeit zur Entwicklung von Morbus Crohn berechnet.

Im Rahmen des neuen Verfahrens kann die Detektion der SNPs mit verschiedenen, dem Fachmann bekannten Verfahren durchgeführt werden. Insbesondere ist hierbei bevorzugt, wenn die Detektion der SNPs im vorliegenden Verfahren mittels MassARRAY®-Technik, Sequenzierung oder einer Hybridisierungs-Technik, beispielsweise einem DNA-, RNA- oder Proteinchip erfolgt. Diese Verfahren weisen jeweils individuelle Vorteile auf.

So ist es mit dem "MassARRAY®"-Verfahren möglich, vergleichsweise kostengünstig große Mengen von SNPs spezifisch zu detektieren. Die Sequenzierung hingegen bietet die Möglichkeit, SNPs in den individuellen genetischen Hintergrund der jeweiligen 3' und 5' flankierenden Sequenzen einzuordnen. Die Hybridisierungstechniken bieten die Möglichkeit, eine Vielzahl von SNPs simultan zu untersuchen. Weiterhin sind diese Techniken hoch etabliert.

Die in dem erfindungsgemäßen Verfahren einzusetzenden Detektionsmethoden können dabei eingesetzt werden, um die Polymorphismen in einer biologischen Probe in intakten Zellen zu detektieren (bspw. *in situ* Hybridisierung), oder in extrahierter DNA (bspw. durch Southern Blot Hybridisierungen). Bei der Allel-spezifischen Hybridisierung können dabei Sonden oder Primer eingesetzt werden, die die Polymorphismus-Stelle überlappt und ca. 5, 10, 20, 25 oder 30 Nucleotide um den SNP herum aufweist.

In einem bevorzugten Verfahren werden mehrere Sonden, die spezifisch an die Allel-Varianten binden können, auf einem festen Träger, d.h. einem Chip, immobilisiert. Eine Beschreibung von Chips, die zur Detektion von Mutationen eingesetzt werden, findet sich bspw. in Warrington et al., "New Developments in High-Throughput Resequencing and Variation Detection Using High Density Microarrays.", Human Mutation (2002): 19:402-409. Ein die entsprechenden allelischen Varianten von zumindest einer polymorphen Genregion der aufgeführten Gene enthaltender Chip wird zur Detektion mit den zu untersuchenden Nucleinsäureproben in Kontakt gebracht, wonach die Identität der allelischen Varianten über einfache Hybridisierungsverfahren ermittelt werden kann.

In einer Ausführungsform des erfindungsgemäßen Verfahrens kann das PCR (Polymerase-Ketten-Reaktions-) Verfahren eingesetzt werden, und zwar unter Verwendung spezifischer Primer, die die Sequenzen von Interesse flankieren. Bei diesem Verfahren werden zunächst aus einer biologischen Probe eines Patienten/Subjekts Nucleinsäuresequenzen isoliert, diese mit den Primern kontaktiert, um eine Region, die den entsprechenden SNP enthält, zu amplifizieren. Das PCR-Produkt durchläuft anschließend mit einem den SNP flankierenden dritten Primer eine zweite PCR Reaktion, welche schlussendlich Rückschlüsse auf das vorhandene Allel in der SNP Position zulässt.

Beispielhaft wird auf das Standardwerk von Sambrock et al., "Molecluar Cloning: A Laboratory Manual", Cold Spring Harbor Laboratory Press, 1989, verwiesen, in dem zahlreiche Verfahren zur Isolierung, Anreicherung und Identifizierung von Sequenzen von Interesse beschrieben sind.

Die Primer, die gemäß der Erfindung zur Detektion der SNP eingesetzt werden, sind lang genug, um spezifisch an die Ziel-Nucleotidsequenz zu hybridisieren. In bestimmten Ausführungsformen kann es darüber hinaus bevorzugt sein, wenn die Primer eine Markierung aufweisen, das bspw. ein Radiosiotop, eine fluoreszierende Verbindung, Enyzme oder Ähnliches sein kann. Die eingesetzten Primer können auch dahingehend modifiziert sein, dass ihre Stabilität erhöht wird, bspw. über den Zuckerrest oder das Phosphat-Rückgrat.

Der Ausdruck "hybridisieren unter stringenten Bedingungen", wie er vorliegend verwendet wird, soll sich auf Hybridisierungsbedingungen von Primern an spezifische Ziel-Sequenzen beziehen, unter welchen keine Kreuz-Hybridisierungen von Polynucleotiden an unverwandte Polynucleotide möglich sind; vorzugsweise beziehen sich die stringenten Hybridisierungsbedingungen auf eine Inkubation bei 42°C in 50% Formamid/10X SSC, gefolgt von drei Waschschritten in 1X SSC/0,2 % SDS, 01,X SSC/0,2 % SDS und 0,1X SSC.

Dem Fachmann sind eine ganz Reihe von Techniken und Verfahren bekannt, mit denen das Vorliegen bestimmter SNPs in den aufgeführten Kandidatengenen detektiert werden können.

Daher betrifft die vorliegende Erfindung auf ein Kit mit einem Primer oder einer Sonde, mit welchen die jeweilige Position der hierin aufgeführten SNP detektiert oder amplifiziert werden kann, nämlich jeweils die Position 101 einer Nucleinsäuresequenz, die ausgewählt ist aus der Gruppe bestehend aus SEQ-ID-Nr. 10, SEQ-ID Nr. 19, SEQ-ID-Nr. 20, oder SEQ-ID-Nr. 21.

Der Kit kann darüber hinaus auch Detektionsmittel und - reagenzien enthalten, wie bspw. Reagenzien für die Hybridisierung von Allel-spezifischne Oligonucleotiden, für die Sequenz-spezifische Amplifizierung, für einen Nucelase-Verdau, eine Sequenzierung, Primer-Extensions-Reaktionen, Größenbestimmungen, u.a.

Die Erfindung betrifft ferner die Verwendung der hierin aufgeführten SNPs, insbesondere des SNP mit der Nummer rs2302685, zur Bestimmung der Prädisposition eines Lebewesens zur Entwicklung einer Crohn's Erkrankung des Ileums mit frühzeitigem Ausbruch.

Zur Bestimmung der Prädisposition einer Person/eines Subjekts, Morbus Crohn zu entwickeln, wird der Polymorphismus mit den hierin aufgeführten SNPs in biologischen Proben der Person/des Subjekts detektiert und mit Kotrollproben ins Verhältnis gesetzt, wodurch die Häufigkeit des Allels in Relation gesetzt werden kann.

Mit der vorliegenden Erfindung werden daher erstmals prädiktive Verfahren und Möglichkeiten bereitgestellt, die auf der Identifizierung der hierin aufgeführten SNPs basieren, die mit der Wahrscheinlichkeit assoziiert sind, dass ein Subjekt Morbus Crohn entwickelt. Die hieraus gewonnene diagnostische Information kann dazu eingesetzt werden, um zu bestimmen, wie das betreffende Subjekt gezielt und rechtzeitig behandelt werden kann, um die Krankheit zu verhindern, zu lindern oder zu behandeln, oder um den Ausbruch der Krankheit hinauszuzögern.

Erfindungsgemäß können die SNPs, die gemäß der vorliegenden Erfindung identifiziert wurden, alleine oder in Kombination zur Bestimmung der Prädisposition eines Lebewesens zur Entwicklung von Morbus Crohn eingesetzt werden.

Weitere Vorteile des neuen Verfahrens ergeben sich aus der Beschreibung und den beigefügten Zeichnungen. Es versteht sich, dass die vorstehend genannten und die nachstehend noch zu erläuternden Merkmale nicht nur in den jeweils angegebenen Kombinationen sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt, von denen:
- Fig. 1a: eine Karte der genomischen Region des TCF4-Gens mit eingezeichneter Lokalisierung der SNPs gemäß der vorliegenden Erfindung zeigt;
- Fig. 1b: eine Karte der genomischen Region des TCF4-Gens mit eingezeichneter Lokalisierung von weiteren SNPs zeigt (oben); die Allelhäufigkeit der genetischen Varianten ist in (unten) im Säulendiagramm gezeigt.
- Fig. 2: eine Karte der genomischen Region des LPR5-Gens mit eingezeichneter Lokalisierung der SNPs zeigt;
- Fig. 3: eine Karte der genomischen Region des LPR6-Gens mit eingezeichneter Lokalisierung der SNPs zeigt;
- Fig. 4: eine zusammenfassende, schematische Übersicht über die Auswirkungen genetischer Veränderungen in bestimmten Genen auf die Paneth-Zelle;
- Fig. 5: die Nucleinsäuresequenzen der LRP6-SNP gemäß der vorliegenden Erfindung zeigt;
- Fig.6: die Nucleinsäuresequenzen der Tcf4-SNP gemäß der vorliegenden Erfindung zeigt; und
- Fig. 7: die Nucleinsäuresequenzen der LRP5-SNP gemäß der vorliegenden Erfindung zeigt.

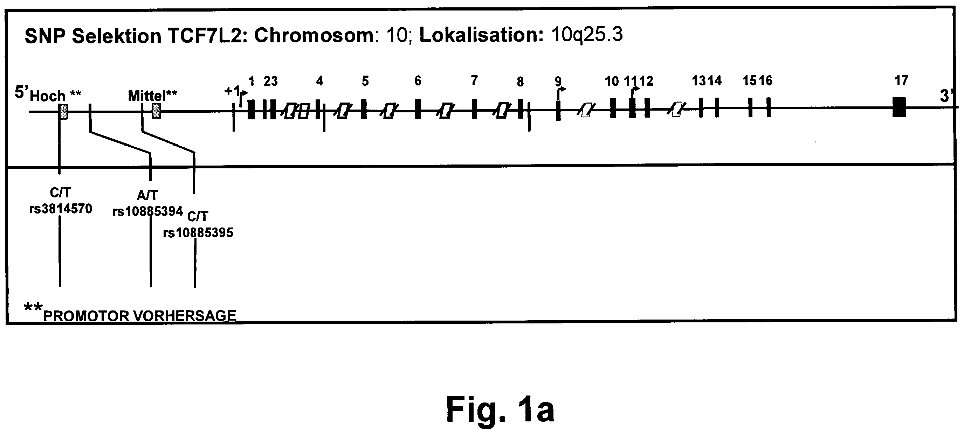

In Fig. 1a ist die Genkarte von TCF4 gezeigt, wobei die Positionen der identifizierten SNPs angezeigt sind. Mit der Nummer "1" ist der SNP1 mit der rs-Nummer rs3814570 bezeichnet, mit "2" der SNP mit der rs-Nummer rs10885394 und mit "3" der SNP mit der rs-Nummer rs 10885395.

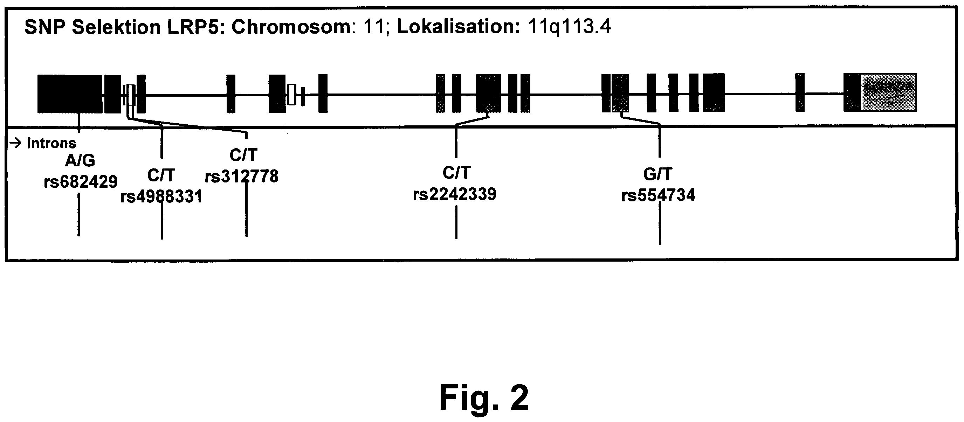

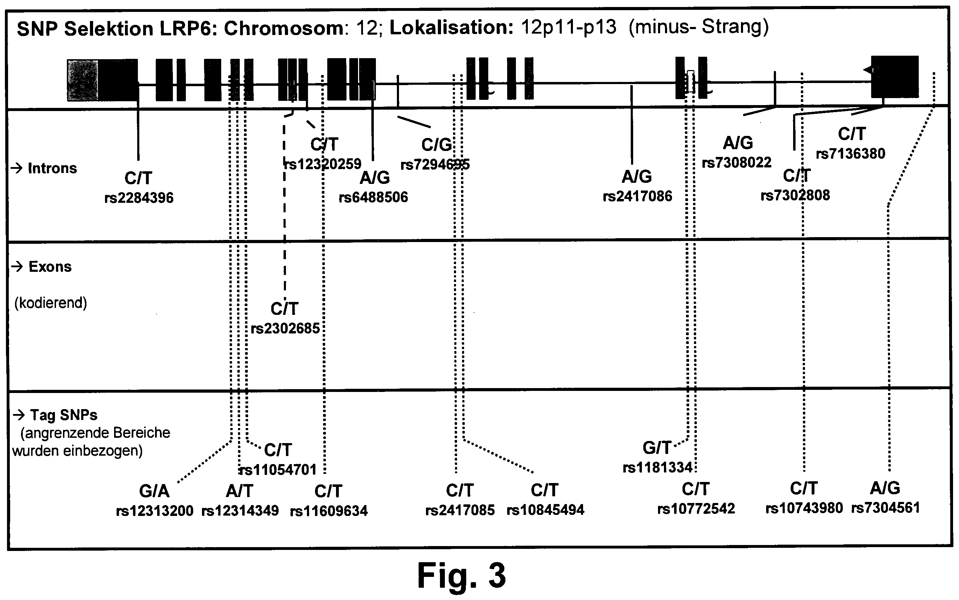

In den Fig. 2 und 3 sind jeweils die Genkarten von LPR5 und LPR6 gezeigt, wobei die jeweiligen Positionen der SNPs in diesen Genen entsprechend angegeben sind.

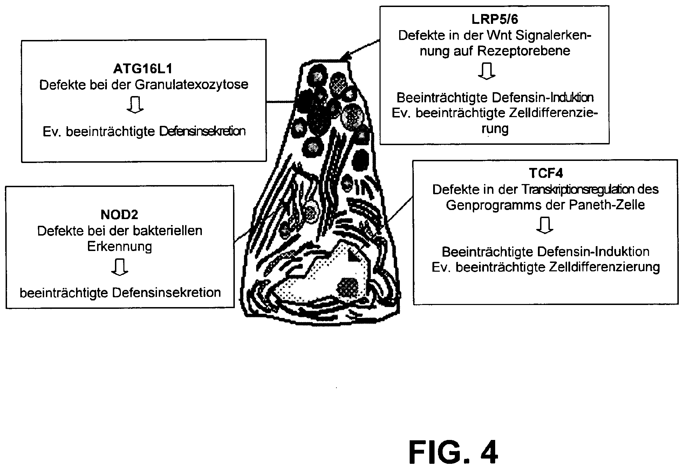

Fig. 4 zeigt eine schematische Darstellung der Auswirkungen von genetischen Veränderungen auf die Paneth-Zelle. Wie hier dargestellt ist, führt eine genetische Veränderung in LPR5/6 zu Defekten in der Wnt-Signalerkennung, was wiederum zu einer Verschlechterung der Induktion der Defensine führt, und möglicherweise auch zu einer beeinträchtigten Zelldifferenzierung. Ferner ist dargestellt, dass genetische Mutationen in TCF4 zu Defekten in der Transkriptionsregulation des Genprogramms in Paneth-Zellen führt, was wiederum zu einer deutlichen Verschlechterung der Induktion von Defensinen, und möglicherweise ebenfalls zu einer beeinträchtigten Zelldifferenzierung, führt.

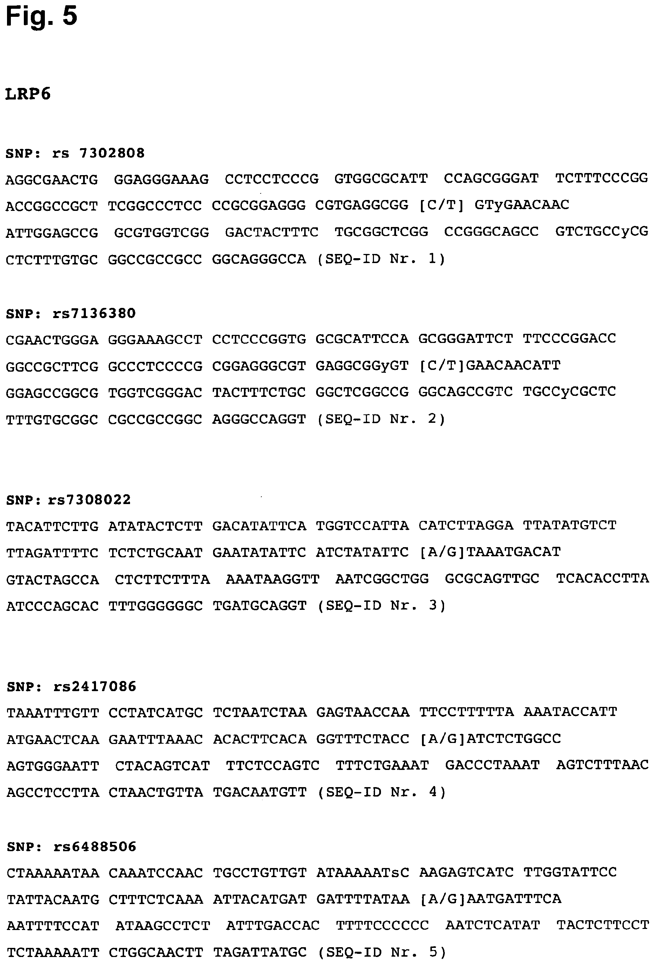

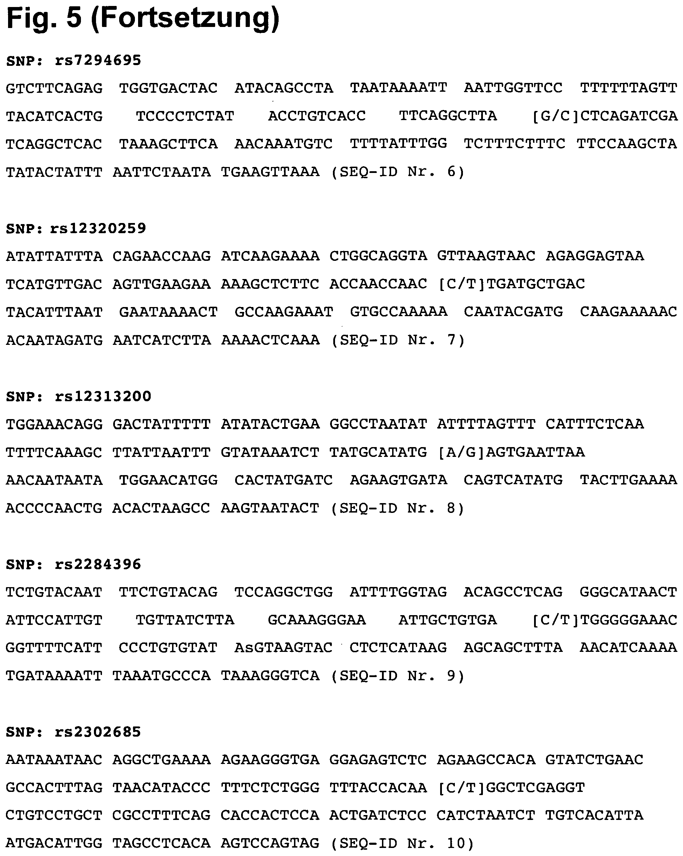

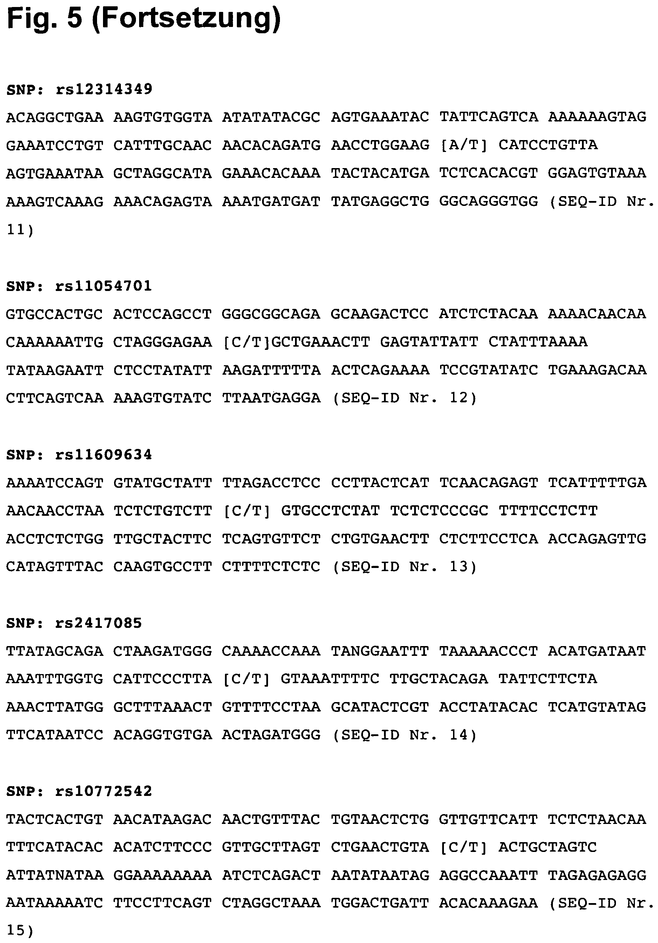

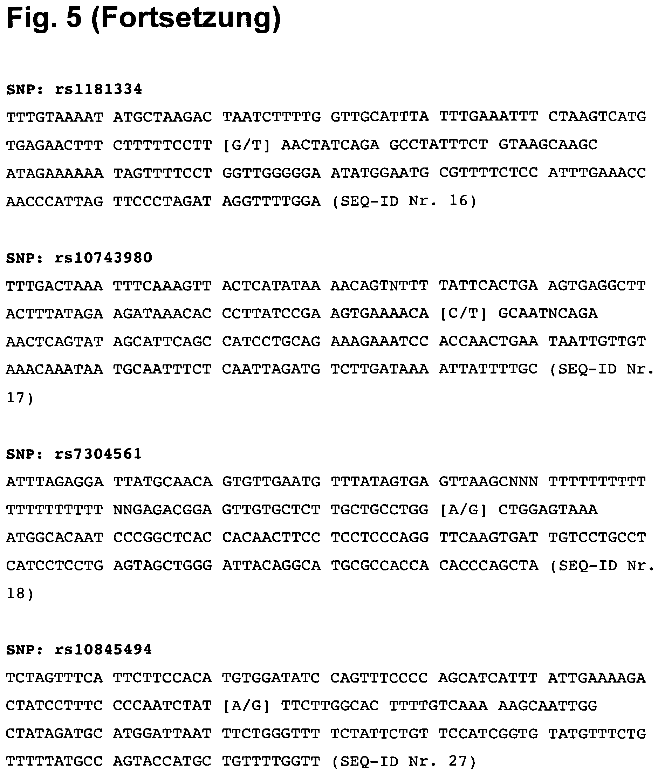

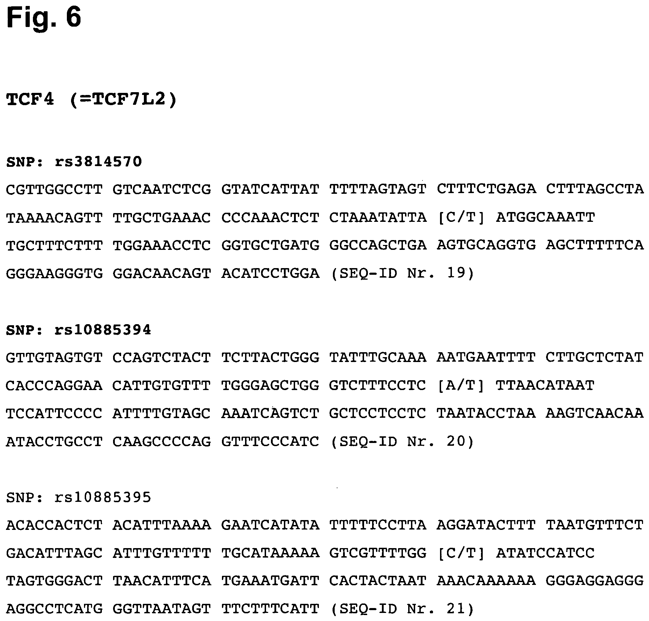

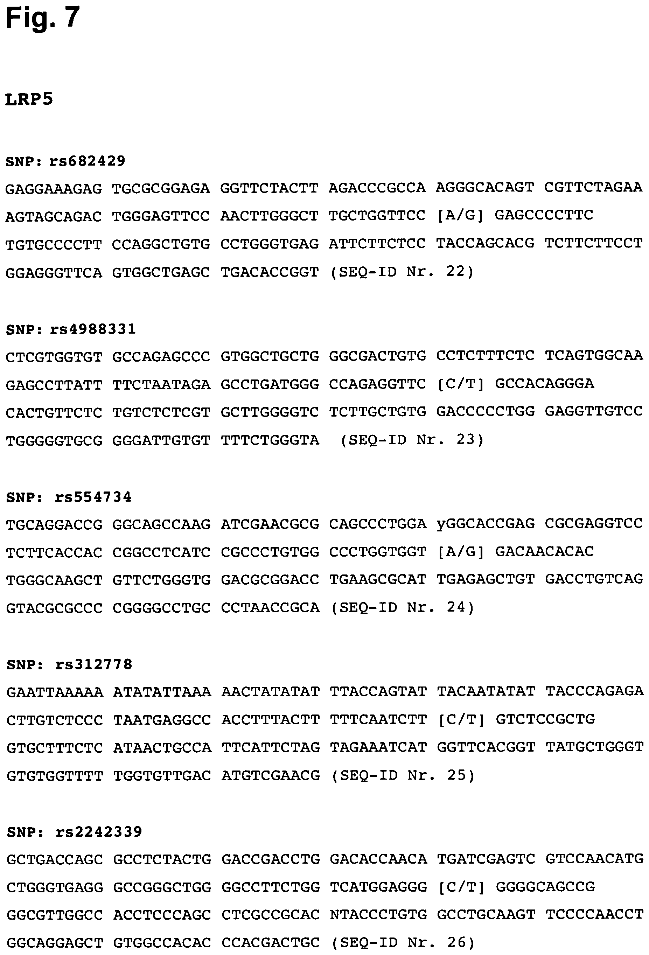

In den Fig. 5 bis 7 ist die Position der erfindungsgemäßen SNP im Kontext der sie umgebenden Nucelinsäuresequenzen gezeigt, wobei in Fig. 5 entsprechend die SNP in LRP6, in Fig. 6 die SNP in Tcf4 und in Fig. 7 die SNP in LRP5 dargestellt sind.

### Beispiel 1

### Material und Methoden

### a) Patienten und humane Materialien

Zur genetischen Analyse wurden von verschiedenen Patientenkohorten DNA-Proben genommen: Patienten kaukasischer Abstammung mit Morbus Crohn (N=259) oder ulcerativer Colitis (N= 149) aus dem Universitätsklinikum Wien; mit Gruppe 1 nicht verwandte gesunde Blutspender kaukasischer Abstammung aus Stuttgart (N=833). Für weitere Tests wurden aus folgenden Patientengruppen DNA-Proben gesammelt: Patienten kaukasischer Abstammung mit Morbus Crohn (N=277) oder ulcerativer Colitis (N=74) sowie gesunde Spender (N=242) der Universität Leuven, Belgien; eine dritte Kohorte von Patienten kaukasischer Abstammung mit Morbus Crohn (N=473) oder ulcerativer Colitis (N=562) sowie gesunder Spender (N=324) aus Oxford. Gemäß der Klassifikation von Montreal wurden drei Untergruppen für Patienten mit Morbus Crohn definiert: Patienten mit Erkrankung ausschließlich des Dünndarms (L1), Patienten mit ausschließlicher Erkrankung des Dickdarms (L2), Patienten mit Erkrankung sowohl des Dünn- als auch des Dickdarms (L3) sowie Patienten mit zusätzlicher Erkrankung des oberen gastrointestinalen Traktes. Zur Bewertung der Häufigkeitsverteilung bestimmter SNPs wurden insgesamt Proben von 225 Patienten mit Morbus Crohn ausschließlich im Dickdarm (L2), 784 Morbus Crohn-Patienten mit zumindest teilweiser Beteiligung des Dünndarms (L1 + L3) sowie 785 Patienten mit ulcerativer Colitis und insgesamt 1399 zufällig ausgewählten Spendern zur Kontrolle ausgewertet. Um größere Unterschiede zwischen den Gruppen bezüglich Alters- und Geschlechts-Zusammensetzung auszuschließen, wurde bei Morbus Crohn Patienten wie Kontroll-Patienten eine weitere Unterteilung gemäß dieser zwei Kriterien vorgenommen.

Weiterhin wurde die Ausprägung und Aggressivität des Krankheitsverlaufs im Einzelfall untersucht. Hierzu wurde bei den Patienten weiterhin überprüft, ob und in welcher Häufigkeit eine inflammatorische, strikturierende oder penetrierende Ausprägung der Krankheit auftrat. Auch der Zusammenhang mit Morbus Crohnbedingten chirurgischen Eingriffen wurde untersucht.

Die Studie wurde durch die Ethikkomitees der Medizinischen Universität Wien, Österreich, des Universitätskrankenhauses Tübingen, Deutschland, der Universität Leuven, Belgien und dem Oxford Radcliffe Hospital Trust genehmigt. Alle Patienten haben nach eingehender Aufklärung eine schriftliche Einverständniserklärung zur Analyse ihrer DNA im Rahmen der vorliegenden Studie gegeben.

### b) Sequenzierung des Tcf-4 Promotors und der Tcf-4 Genregion

Um mögliche genetischen Varianten im *Tcf-4* Promotor zu bestimmen, wurde die 2.1 kb upstream Region zufällig ausgesuchter gesunder Patienten (n=10) sowie die von Patienten mit Morbus Crohn im Dünndarm (n=10) sequenziert. Darüber hinaus wurde die Rgion des *Tcf-4* Gens sequenziert, in welchem in der Literatur funktionelle Insertionen und Deletionen bei Darmkrebs beobachtet wurden. Anschließend wurde eine Sequenzanalyse bekannter *Tcf-4* Exone durchgeführt, einschließlich ∼100 bp flankierenden Introns, um zusätzlich mögliche Varianten dieses Gens in diesen Regionen zu identifizieren. Die Primer wurden unter Einsatz von ENSG00000148737 der "Ensemble genome browser" Datenbank für die Sequenzierung der Promotoren und der Exons ausgewählt. Die Sequenzierung wurde nach Standardverfahren durchgeführt.

### c) Genotypisierung

Leukozyten-DNA wurde gemäß eines Standardverfahrens (QIAamp DNA Blood Mini Kit, Qiagen, Hilden, Deutschland) aus Vollblutproben isoliert. Für die Genotypisierung wurde die matrix assisted laser desorption/ionization time-of-flight (MALDI-TOF) basierte massenspektrometrische (MS) Analyse allelspezifischer Primerextension-Produkte in einem System von Bruker (Daltonik, Leipzig, Deutschland) verwendet. In einer Untergruppe von Proben wurde das mittels MALDI-TOF MS detektierte Vorhandensein von SNPs durch TaqMan®-Analyse und direkte Sequenzierung überprüft. Die MALDI-TOF MS basierte Genotypisierung von DNA-Proben wurde mittels des MassARRAY® Compact Systems von Sequenom (San Diego, USA) durchgeführt.

### d) Computeranalyse und Statistiken

Unter Verwendung des "Promoter 2.0: zur Erkennung von PolII-Promotersequenzen" wurde ein *in silico-*Screen einer 10 kb *Tcf-4* upstream-Region durchgeführt. Die TESS (Transcriptions-Element-Suchen-System)-Datenbank-Software ermöglichte es, potenzielle Bindestellen für bestimmte Transkriptionsfaktoren in den potenziell interessanten Sequenzen zu untersuchen. Unter Verwendung der Finetti-spezialisierten Software (http.//ihg2.helmholtz-muenchen.de/cgi-bin/hw/hwa1.pl) wurden die Polymorphismen hinsichtlich des Hardy-Weinberg-Gleichgewichts getestet, indem in den drei Kohorten der Log-Likelihood-Ratio-/Chi-Quadrat-Test angewandt wurde. Zur genetischen Analyse (Vergleich der IBD-Untergruppen gegenüber Kontrollen) wurde diese Software eingesetzt, um ungerade Verhältnisse, Konfidenzintervalle (C.I.) zu berechnen, und um "Pearson's Goodness-of-fit Chi-Quadrat-Tests" durchzuführen. Die Unterschiede in den Genotyphäufigkeiten waren Gegenstand von sowohl *t*-Tests und Armitage-Trend-Tests. Werte unter 0,05 wurden als signifikant betrachtet. Das Kopplungsungleichgewicht ("linkage disequilibrium") zwischen *Tcf-4* SNPs und den Haplotyp-Blöcken wurde berechnet und unter Einsatz von Haploview identifiziert. Um eine zufällige Assoziierung des SNP rs3814570 auszuschließen, wurde die Signifikanz der p-Werte < 0,05 unter Verwendung der Korrektur nach Benjamini-Hochberg in der Gesamtgruppe verifiziert.

### e) NOD2-Genotypanalyse

Die Genotypisierung der üblichen NOD2-Varianten (SNP8, SNP12 und SNP13) wurde in den Proben der Wiener Patienten unter Durchführung der "TaqMan"-Technologie (Applied Biosystems, Foster City, Kalifornien, USA), wie zuvor beschrieben (9) durchgeführt.

### Beispiel 2: Tcf-4

### a) SNP-Auswahl und Haplotypen

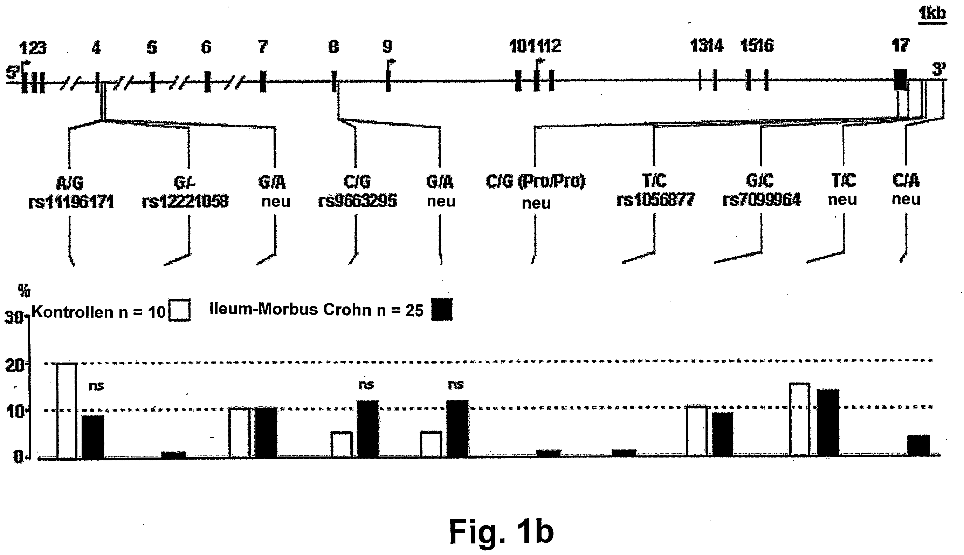

Um die mögliche genetische Kopplung von *Tcf-4* mit Ileum-CD zu untersuchen, wurden SNPs hinsichtlich der Sequenzierung von 2,1 kb der 5'-flankierenden Region von *Tcf-4* gescreent, und zwar in einer zufälligen Gruppe von 10 Ileum-CD-Patienten und 10 gesunden Kontrollen. In dieser angenommenen Promoterregion (siehe Fig. 1) wurden acht SNPs gefunden, von welchen drei (rs3814570, rs10885394, rs10885395) in Kopplungsungleichgewicht ("Linkage Disequilibrium", (LD)) in sowohl der Patienten- als auch der Kontrollgruppe waren. In der Kontrollgruppe waren zwei von zehn Individuen hinsichtlich dieser Varianten heterozygot; bei Patienten mit Ileum-CD waren sechs von zehn Individuen heterozygot. Basierend auf diesen Ergebnissen wurde eine gut untersuchte Kohorte von Patienten mit CD sowie gesunden Kontrollen aus Wien (Österreich) untersucht. Sowohl in der Kontrolle als auch in der CD-Gruppe wurde ein LD zwischen den drei SNPs gefunden, über welches ein neuer Haplotyp-Block definiert wurde (siehe Fig. 2).

Eine *in silico*-Analyse des Promoters und der Transkriptionsfaktoren-Bindestellen der sequenzierten Region ergab eine potenzielle regulatorische Region nahe der Lokalisierung von rs3814570. Aufgrund der (i) beobachteten verminderten Expression der *Tcf-*4 mRNA, (ii) der höheren Häufigkeit der Promotervarianten und aufgrund (iii) des Vorliegens eines angenommenen regulatorischen Lokus wurde die Hypothese getestet, dass rs3814570 eine höhere Häufigkeit bei Patienten mit CD des Dünndarms zeigt. Um zusätzliche größere Varianten in der Genregion und mögliche LD der identifizierten Promoter-SNPs mit anderen potenziell funktionellen Varianten im *Tcf-*4-Gen auszuschließen, wurden bekannte codierende Exons sequenziert, mit ungefähr 100 kb überlappenden Introngrenzen in 10 zufällig ausgewählten Kontrollen (6 identisch zur Promoteranalyse) sowie in 25 Patienten mit Ileum-CD (7 identisch zur Promoteranalyse). Zehn zusätzliche angenommene SNPs wurden gefunden, von welchen zwei in LD waren, jedoch zeigte keines ein LD mit den beschriebenen Promoter-SNPs. Eine weitere Suche für Haplotypen in *Tcf-4* wurde basierend auf den vom HapMap-Projekt veröffentlichten Daten durchgeführt, und es konnte kein Haplotyp-Block identifiziert werden, der rs3814570 oder zusätzliche SNPs in der Genregion einschloss.

### b) Eine Tcf-4-Promoter-Variante ist mit der Ileum-CD-Prädisposition assoziiert

Die Analyse der Häufigkeitsverteilung des SNP rs3814570 wurde in insgesamt 1399 Kontrollen (T-Allel-Häufigkeit = 25,59 %) durchgeführt, sowie in 785 UC-Patienten (T-Allel-Häufigkeit = 25,22 %), 225 CD-Patienten mit L2-Klassifizierung (T-Allel-Häufigkeit = 24, 67 %) und 784 CD-Patienten mit Ileum-Beteiligung (L1 + L3) (T-Allel-Häufigkeit = 29,66 %). Im Gegensatz zu UC (OR 0,981, 95 % CI 0,851 bis 1,131, n.s., was ähnlich zu den Kontrollen war) zeigten die CD-Patienten zusammengefasst eine schwache Assoziierung für die Variante (T-Allel-Positivität: OR 1,182, 95 % CI 1,005 bis 1,391, p = 0,04358).

**Tabelle 3: Übersicht über die Herkunft der Proben**

| | **Kontrollen** | **UC** | **CD (L1)** | **CD (L2)** | **CD (L3)** |
|---|---|---|---|---|---|
| Wien | 833* | 149 | 54 | 55 | 150 |
| Leuven | 242 | 74 | 81 | 45 | 151 |
| Oxford | 324 | 562 | 94 | 125 | 254 |

| | | | | | |
|---|---|---|---|---|---|
| *gesunde Blutspender aus Stuttgart | | | | | |

In Übereinstimmung mit der Anfangshypothese zeigte die Untersuchung der unterschiedlichen CD-Subgruppen eine Assoziierung der kleineren Variante (T) mit Ileum-CD (OR 1,226, 95 % CI 1,068 bis 1,407, p = 0,00371), jedoch nicht mit Dickdarm-CD (OR 0,952, 95 % CI 0,756 bis 1,199, n.s.). Der Test hinsichtlich der Allelen-Positivität durch Analyse von Wildtyp-Homozygoten Individuen (CC) gegenüber sämtlichen Trägern der kleinen Variante (CT + TT) zeigte einen deutlicheren Effekt bei Vergleich der gesunden Kontrollen gegenüber Ileum-CD (OR 1,271, 95 % CI 1,066 bis 1,515, p = 0,007372).

Da es Unterschiede in den Allel-Häufigkeiten zwischen den unterschiedlichen Kohorten gab, wurde überprüft, ob diese offensichtlichen Häufigkeitsunterschiede statistisch signifikant waren. Im Allgemeinen zeigte die Oxford-Kohorte eine niedrigere T-Allel-Häufigkeit in Kontrollen (23,30 %) im Vergleich zur Leuven-Kohorte (26,65 %) sowie zur Wien-Kohorte (26,17 %). Das Gleiche galt für CD-Patienten (T-Allel-Häufigkeit in Oxford: 27,38 %, Leuven: 30,14 % und Wien: 28,96 %), konnte jedoch teilweise durch den unterschiedlichen Prozentsatz an Dickdarm-CD-Patienten in den Gruppen erklärt werden. Für CD mit lediglich Ileum-Beteiligung ähnelten sich die Häufigkeits-Verteilungen in den Kohorten eher (T-Allel-Häufigkeit in Oxford: 28,30 %, Leuven: 30,82 % und Wien: 30,64 %) und waren nicht signifikant unterschiedlich. Obgleich wir eine mögliche Veränderung in der Häufigkeitsverteilung zwischen der Oxford-Kontrollgruppe mit sowohl den Leuven- (Allel-Häufigkeit: OR 1,196, 95 % CI 0,912 bis 1,569, p = 0,19618) und Wien-Kontrollen (Allel-Häufigkeit: OR 1,167, 95 % CI 0,943 bis 1,443, p = 0,15453) gefunden haben, waren die Unterschiede nicht signifikant. Daher wurde eine erhöhte SNP-Häufigkeit in Ileum-CD-Patienten in drei unabhängigen europäischen Kohorten gezeigt, und es konnte eine eindeutig signifikante Assoziierung der kleinen Variante für rs3814570 mit Ileum-CD in der kombinierten Analyse aller Proben beobachtet werden.

### c) Die Assoziierung von rs3814570 mit Ileum-CD ist unabhängig vom Geschlecht, jedoch etwas mehr ausgeprägt in Patienten > 40 Jahre

Um sicherzugehen, dass es keine Desorganisation bezüglich des Alters als auch des Geschlechts gibt, wurden sämtliche Kontrollen ebenso wie die CD-Patientengruppe gemäß diesen Kriterien untergruppiert. Es gab keine übereinstimmenden Unterschiede bezüglich der Allel-Häufigkeit zwischen Männern und Frauen in sowohl den Kontrollen als auch den Patienten; daher wurde ein geschlechtsspezifischer Effekt der Variante ausgeschlossen. Interessanterweise wurde eine erhöhte Assoziierung der Variante im Vergleich von Patienten mit Ileum-CD, jedoch nicht bei ausschließlicher Dickdarm-CD, der Altersgruppe A3 (> 40 Jahre) mit Kontrollen der gleichen Altersgruppe in der Gesamtanalyse gefunden, ebenso wie in zwei getrennten Kohorten (Leuven und Oxford). In der Gesamtanalyse lag eine statistische Signifikanz für homozygote Träger vor (homozygote Träger: OR 2,023, 95 % CI 1,010 bis 4,052, p = 0,04347).

### d) Rs3814570 zeigt die höchste Häufigkeit in Patienten mit strikturierender Ileum-Crohn's Erkrankung

Die Patienten wurden gemäß deren Auftreten in B1 (entzündlich), B2 (strikturierend) und B3 (durchdringend) eingruppiert. Die höchste Häufigkeit wurde in der Gesamtanalyse innerhalb der Ileum-CD-Untergruppe mit strikturierendem Auftreten (T-Allel-Häufigkeit: 31,25 %) gefunden. Dies war ebenso in zwei getrennten Kohorten offensichtlich (T-Allel-Frequenz in Oxford: 29,81 %, Leuven: 35,83 %), nicht jedoch in L2-CD-Patienten. Die Assoziierung des SNP mit strikturierender Ileum-CD im Vergleich mit gesunden Kontrollen zeigte eine hohe Signifikanz in der Gesamtanalyse (Allel-Häufigkeit: OR 1,322, 95 % CI 1,079 bis 1,619, p = 0,00686), und es wurde eine zusätzlich erhöhte Menge an homozygoten Trägern beobachtet (homozygote Träger: OR 1,708, 95 % 1,107 bis 2,634, p = 0,01460). Um eine mögliche Assoziierung mit der Aggressivität der Krankheit zu identifizieren, wurden die Patienten in solche eingruppiert, die zumindest einen chirurgischen Eingriff hinsichtlich CD hatten, und solche, die keinen hatten. Es wurden keine übereinstimmenden Ergebnisse beobachtet; obgleich in zwei Kohorten ein Trend hinsichtlich einer höheren Häufigkeit in der Ileum-CD-Gruppe mit chirurgischem Eingriff (T-Allel-Häufigkeit in Oxford: 28,93 % und Leuven: 31,58 %) und eine signifikante stärkere Assoziierung mit Ileum-CD in der Gruppe mit chirurgischem Eingriff im Vergleich zu Kontrollen in einer Kohorte (Oxford-Allel-Frequenz: OR 1,340, 95 % CI 1,030 bis 1,742, p = 0,02885) vorlag.

### e) Rs3814570 verleiht das Risiko eines zusätzlichen L4-Phänotyps in Patienten mit Ileum-CD

Um die Frage nach einer oberen GIT-Beteiligung (L4) spezifisch anzusprechen, wurden die Patientengruppen in zwei weitere Untergruppen gemäß diesem spezifischen zusätzlichen Phänotyp aufgeteilt. Ganz allgemein war die Menge an Patienten mit oberer GIT-Beteiligung recht niedrig: Leuven-Patienten mit zusätzlichem L4-Phänotyp: 12 Patienten L3; 4 Patienten L2; 6 Patienten L1; Oxford-Patienten mit zusätzlichem L4-Phänotyp: 36 Patienten L3; 4 Patienten L2; 10 Patienten L1; Wien-Patienten mit zusätzlichem L4-Phänotyp: 40 Patienten L3; 10 Patienten L2; 11 Patienten L1. Beim Vergleich der Allel-Häufigkeiten mit den Kontrollen konnten wir einen leichten Anstieg bei den Patienten mit Ileum-CD mit einem zusätzlichen L4-Phänotyp (T-Allel-Häufigkeit: 32,17 %) finden. Dies trug jedoch nicht für die L2-Patienten mit oberer GIT-Beteiligung bei. Die stärkere Assoziierung der seltenen Variante war also in der Gesamtanalyse statistisch signifikant (Allel-Häufigkeit: OR 1,379, 95 % CI 1 1,033 bis 1,842, p = 0,02882).

### f) Rs3814570 ist unabhängig von NOD2

Unter der Voraussetzung, dass die 1007fsinsC-Mutation (SNP13) in *NOD2* ein bekannter Suszeptibilitäts-Faktor für CD des Ileums ist und dass sie mit reduzierten HD-5 und -6-Leveln assoziiert ist, wurde untersucht, ob die beobachtete Assoziierung von rs3814570 mit Ileum-CD unabhängig von *NOD2* in den Wien- und Leuven-Kohorten war. Es war zuvor berichtet worden, dass die Effekte des reduzierten *Tcf-4* auf Paneth-Zellen- -Defensine in Ileum-CD-Patienten unabhängig von den Effekten der SNP13 NOD2-Variante waren, da diese Patienten mit dieser *NOD2*-Mutation eine deutlich ausgeprägtere Reduzierung der HD-5 und -6-Expression zeigten (40). Die Unabhängigkeit von diesen Faktoren legt nahe, dass der Ausschluss von Patienten, die *NOD2* SNP13 tragen, ähnliche Allel-Häufigkeiten von rs3814570 bei den verbleibenden Ileum-CD-Patienten erzielen sollte. Beim Vergleich sämtlicher Leuven-Ileum-CD-Patienten (n = 232) mit einer Untergruppe, bei dem Patienten mit SNP13 (n = 191) ausgeschlossen waren, gab es tatsächlich keine Unterschiede in der Allel-Häufigkeit (OR 0,991) oder Allel-Positivität (OR 0,984). Gleiches galt für die Wien-Ileum-CD-Patienten (ns = 204): nach SNP13-Ausschluss (n = 154) ergab die Allel-Häufigkeit einen OR von 1,057 und eine Allel-Positivität von OR 1,040. Daher ändert der Ausschluss von Patienten mit der *NOD2*-Frameshift-Mutation SNP13 nicht die beobachteten Allel-Häufigkeiten von rs3814570 in Patienten mit Ileum-CD, was die unterschiedlichen Effekte dieses *Tcf-4*-SNP und *NOD2* SNP13 bei Ileum-CD unterstützt.

Mit den erzielten Ergebnissen konnte gezeigt werden, dass mit dem neuen Verfahren eine hohe Prognosewahrscheinlichkeit für Morbus Crohn erreicht werden kann. Weiterhin zeigen die Ergebnisse der oben aufgeführten statistischen Analysen, dass es mit dem neuen Verfahren möglich ist, basierend auf dem Vorhandensein oder Nichtvorhandensein verschiedener SNPs, insbesondere im 5'-UTR des TCF-4 Gens, eine Wahrscheinlichkeit für das Auftreten bestimmter Ausprägungen einer Erkrankung an Morbus Crohn vorherzusagen.

Die vorliegenden Ergebnisse zeigen, dass Gene, die wichtige Funktionen im Wnt Signalweg einnehmen, Veränderungen im Zusammenhang mit der Erkrankung zeigen. Da der Signalweg besonders wichtig für die einwandfreie Entwicklung der Paneth-Zelle ist, sind dabei beteiligte Faktoren ausgezeichnete Zielobjekte in der Untersuchung der gestörten Funktionen von Panethzellen. Die zur Verfügung stehenden Daten lassen eindeutig darauf schließen, dass Faktoren, die die Funktion von Panethzellen mitbestimmen, wichtige Kandidaten in der Pathogenese von Morbus Crohn des Dünndarms darstellen. Vorliegend sind also genetische Veränderungen, die mit Morbus Crohn des Dünndarms in Zusammenhang stehen, mit dem Nachweis für Tcf-4, dem Wnt Transkriptionsfaktor, der direkt HD-5 und -6 induziert, sowie für Low Density Lipoprotein Receptor-Related Protein LRP5 und LRP6, wichtigen Rezeptormolekülen des Signalwegs, identifiziert worden.

Somit ist das neue Verfahren ein wertvolles Werkzeug zur Prognose des Auftretens von chronisch entzündlichen Darmerkrankungen sowie der individuellen Diagnose der solchen Krankheiten zugrunde liegenden genetischen Defekte.

### Beispiel 3: LRP6

### Material und Methoden

Als Proben wurde das gleiche Material eingesetzt wie in Beispiel 1, und es wurden die gleichen Methoden zu deren Untersuchung verwendet wie für Beispiel 1 beschrieben.

### a) LRP6

Die Auswahl von LRP6 als Kandidaten-Gen basierte auf dessen spezifischem und Gewebe-unabhängigen Abnahme in erkrankten Patienten. Um potentielle mit der Krankheit assoziierte Varianten in der Intronregion sowie der 3'- und 5'-Region in der Analyse abzudecken, wurden SNPs der codierenden Region und Tag SNPs mit eingeschlossen. Die Auswahl von Tag SNPs wurde mittels einem "paarweisem Verfahren" durchgeführt, indem die Tag SNP-Auwahlfunktion der internationalen "HapMap"-Projekt Homepage (http://hapmap.ncbi.nlm.nih.gov/index.html.en) eingesetzt wurde. Das Ziel dieses internationalen HapMap Projekts ist es, die Haplotypen des menschlichen Genoms zu karthographieren. Um auch Promotorvarianten und andere SNPs up- oder downstream von LRP6 mit einzuschließen, wurde außerhalb der Genregion (10% ca. 7.3kb (kb= Kilobasen) herausgezoomt.

### b) LPR6-Genotypisierung

Leukozyten-DNA wrude mittels Standardverfahren isoliert (wie unter Beispiel 1; Material und Methoden, c) beschrieben).

### c) Computer-Analyse und Statistiken

Die mRNA-Expressions-Level unterschiedlicher Faktoren wurden mittels der Graph-Pad Prism Software, Version 4.0 (siehe www.graphpad.com) analysiert. Es wurden entweder nicht-parametrische oder parametrische (im Falle einer Normalverteilung) Analysen durchgeführt, und zwar unter Verwendung der des *U*-Tests von Wilcoxon, Mann und Whitney oder des "Student t-Tests", um die gruppierten Daten zu vergleichen. P-Werte von kleiner als 0.05 wurden als statistisch signifikant betrachtet. Für Korrelations-Tests wurde die "Spearmen-Rank"-Analyse durchgeführt, um Beziehungen zwischen unterschiedlichen Genprodukten zu untersuchen. Mittelwerte werden in den Figuren mit ihren entsprechenden Standardfehlern dargestellt. Unter Verwendung der Finetti-spezialisierten Software (http.//ihg2.helmholtz-muenchen.de/cgibin/hw/hwa1.pl) wurden die Polymorphismen hinsichtlich des Hardy-Weinberg-Gleichgewichts getestet, indem in den drei Kohorten der Log-Likelihood-Ratio-/Chi-Quadrat-Test angewandt wurde. Zur genetischen Analyse (Vergleich der IBD-Untergruppen gegenüber Kontrollen) wurde diese Software eingesetzt, um ungerade Verhältnisse, Konfidenzintervalle (C.I.) zu berechnen, und um "Pearson's Goodness-of-fit Chi-Quadrat-Tests" durchzuführen. Die Unterschiede in den Genotyphäufigkeiten waren Gegenstand von sowohl *t*-Tests und Armitage-Trend-Tests. Werte unter 0,05 wurden als signifikant betrachtet. Das Kopplungsungleichgewicht ("linkage disequilibrium") zwischen *Tcf-4* SNPs und den Haplotyp-Blöcken wurde berechnet und unter Einsatz von Haploview identifiziert.

### d) Die LRP6-Expression ist bei Ileum-CD in einer Gewebe-unabhängigen Weise reduziert

Untersucht wurden die mRNA-Level von LRP5, LRP6, HD-5 und HD-6, Interleukin-8 (IL-8) sowie von GapDH (Glycerinaldehyd-3-phosphat-Dehydrogenase) in Ileum- und Kolon-Biopsieproben (Daten nicht gezeigt). Sämtliche Kontrollen und Patienten besaßen als Genotyp den *NOD2*-Wildtyp, um verzerrte Effekte aufgrund zusätzlich reduzierter Defensin-Level in *Nod2* SNP13 mutierten Individuen zu vermeiden. Bei der GapDH-Expression zeigten sich keine signifikanten Unterschiede zwischen den Gruppen, sowohl in der Ileum als auch der Kolon-Gruppe. Jedoch wurde eine starke und signifikaten Abnahme des LRP6-mRNA-Levels bei CD-Patienten mit einer Beteiligung des Dünndarms beobachtet. Bei den Ileum-Proben war der Effekt sowohl in Patienten zu beobachten, die einen L1-Phänotyp aufweisen (nur Dünndarm), als auch bei Patienten, bei denen zusätzlich der Kolon beteiligt ist (L3); der Effekt war allerdings nicht bei Patienten mit ausschließlicher Kolon-Beteiligung (L2) zu beobachten. LRP5, von dem bekannt ist, dass es allgemein mit LRP6 coexprimiert wird, fehlte diese signifikante Abnahme in der mRNA-Expression. Innerhalb der Proben zeigten die mRNA-Level von LRP6 und HD5 einen hohen Korrelationsgrad. Im Folgenden wurde die LRP6-Expression in Kolon-Proben von gesunden Individuen, von Patienten mit Colitis indeterminata und mit chronisch entzündlicher Darmerkrankung (IBD) untersucht. Im Kolon konnten keine Unterschiede in Bezug auf die Entzündung in der LRP6-Expression detektiert werden. Dies und die Gewebeunspezifische Abnahme des Faktors in dem L1-Subphänotyp spricht gegen einen Sekundär-Effekt im Ileum von Patienten mit Dünndarm-CD.

### e) Eine seltene kodierende Variante von LRP6 ist mit einem Phänotyp mit frühzeitigem Ausbruch bei Ileum-CD assoziiert

Im Weiteren wurden Häufigkeitsverteilungen und Kopplungsungleichgewichte der ausgewählten LRP6 SNPs in einer gut definierten Oxford-Kohorte untersucht, die fast 2000 DNA-Proben mit einschloss, die in gesunde Kontrollen und IBP-Patienten gruppiert waren. Es wurde eine Assoziierung der codierenden seltenen Variante von rs2302685 mit Patienten mit Ileum-CD gefunden, die ganz spezifisch einen Phänotyp mit frühzeitigem Ausbruch aufwiesen. In Übereinstimmung mit diesen Ergebnissen konnte diese spezifische Assoziierung durch nachfolgende Analysen zweier zusätzlicher Kohorten aus Leuven und Wien bestätigt werden. Die nachfolgende Tabelle 4 zeigt eine Übersicht dieser Ergebnisse:

**Tabelle 4: Häufigkeitsverteilung und Genotypen**

| **Übersicht Ileum-CD** | | **Alter bei Diagnose** | | **Krankheits-Verlauf** | | |
|---|---|---|---|---|---|---|
| | | 18 u. älter | bis einschl. 17 | entzündl. | strikutrier. | senetr. |
| Genotypen (n) | CC | 452 | 48 | 98 | 201 | 198 |
| | CT | 228 | 35 | 58 | 108 | 126 |
| | TT | 21 | 10 | 5 | 9 | 16 |
| Proben (n) | alle | 701 | 93 | 161 | 318 | 340 |
| | | | | | | |
| Genotpyen (Häufigkeit) | CC | 64,48% | 51,61% | 60,87% | 63,21% | 58,24% |
| | CT | 32,52% | 37,63% | 36,02% | 33,96% | 37,06% |
| | TT | 3,00% | 10,75% | 3,11% | 2,83% | 4,71% |
| | | | | | | |
| Allel Häufigkeit | C | 80,74% | 70,43% | 78,88% | 80,19% | 76,76% |
| | T | 19,26% | 29,57% | 21,12% | 19,81% | 23,24% |

Eine Kombination aller getesteten Proben zeigte, dass eine Assoziierung beim Ileum-CD-Phänotyp mit einem frühzeitigem Ausbruch der Krankheit und einem penetrierenden Verhalten auf einen Einfluss der Variante auf den Krankheitsverlauf und deren Schwere hinweist, wohingegen das Geschlecht der Patienten keinen Einfluss auf die Häufigkeitsverteilung bei Ileum-CD hatte.

Ferner wurde untersucht, ob eine Dosis-Abhängigkeit der Variante, wie sie in der Darstellung des Effekts zwischen Homozygoten und Heterozygoten dargestellt wird, die Rolle der Variante bei Ileum-CD mit frühzeitigem Ausbruch verstärkt. Es stellte sich heraus, dass dies der Fall ist, da die subphänotypisierte Gruppe eine äußerst erhöhte Menge an homozygoten Trägern mit einschloss, was mit einem signifikant erhöhtem Risiko für den entsprechend homozygotene Genotyp einhergeht.

Um die potentielle Rolle von LRP6 bei der Regulierung der alpha-Defensine-mRNA-Expression in Panethzellen zu untersuchen, wurden deren Level entsprechend dem LRP6 rs2302685 Genotyp in den Patienten untersucht. Hierzu wurden die Patienten und die Kontrollen der Studie genotypisiert und die Proben in LRP6 "Mutante" LRP6 und "Wildtyp" umgruppiert.

Da bekannt ist, dass eine NOD2-Frameshift-Mutation HD5- und HD6-mRNA-Level beeinflusst, wurde auch der Effekt dieser Mutation als vergleich untersucht. Es zeigte sich (Daten nicht gezeigt), dass Ileum-CD-Patienten mit einem hetero- oder homozygoten mutierten LRP6-Genotyp die niedrigsten HD5-Level besaßen. Der Effekt der selten codierenden LRP6-Variante auf die HD-5-mRNA-Expression war vergleichbar, jedoch etwas deutlicher als der Effekt der NOD2 SNP13 Frameshift-Mutation, die zuvor mit verminderten Panethzell-alpha-Defensine-Level in Verbindung gebracht wurde. Die Expressions-Level von LRP6 waren bei Ileum-CD-Patienten erniedrigt, und zwar unabhängig vom *NOD2* oder *LRP6* Genotyp.

Die Ergebnisse hinsichtlich LRP6 zeigen, dass dieser ein neuer und relevanter Wnt-Signalweg-Faktor bei der Ileum-CD ist. In dem betroffenen Ileum von CD-Patienten ist LRP6 auf dem Transkriptionslevel beeinträchtigt. Die Expressionslevel von LRP6 und HD5 korrelierten stark, und eine LRP6-Mutation, die mit einem frühzeitigem Ausbruch der Krankheit assoziiert ist, reduzierte die alpha-Defensin-Expression in Panethzellen bei Ileum-CD weiter. Die genetische Verbindung und die Gewebeunspezifische Abnahme von LRP6 weisen auf eine primäre und außergewöhnliche Funktion des Korezeptors bei der Krankheit hin und bestätigen diesen.

## Patentansprüche

1. Verfahren zur Bestimmung einer Prädisposition eines Lebewesens für die Entwicklung von Morbus Crohn, bei welchem in einer biologischen Probe des Lebewesens das Vorliegen von zumindest einem Einzelnukleotid-Polymorphismus (SNP) bestimmt wird, **dadurch gekennzeichnet, dass** der zumindest eine SNP in mindestens einem Gen bestimmt wird, das für ein mit dem Wnt-Signalweg in Paneth-Zellen assoziiertes Protein kodiert, wobei das zumindest eine Gen TCF4 oder LRP6 ist, und wobei der zumindest eine SNP ausgewählt ist aus einem der folgenden: rs3814570, rs10885394, rs10885395 (TCF 4) und rs2302685 (LRP6).

2. Verfahren nach Anspruch 1, **dadurch gekennzeichnet, dass** die Prädisposition eines Lebewesens für Morbus Crohn des Dünndarms bestimmt wird.

3. Verfahren nach einem der Ansprüche 1 oder 2, in dem die Bestimmung des Vorliegens oder Nichtvorliegens des mindestens einen SNPs durch Primerextension eines PCR-Produkts und MALDI-TOF-Analyse erfolgt.

4. Verfahren nach einem der Ansprüche 1 oder 2, in dem die Bestimmung des Vorliegens oder Nichtvorliegens des mindestens einen SNPs durch Sequenzierung eines PCR-Produkts erfolgt.

5. Verfahren nach einem der Ansprüche 1 oder 2, in dem die Bestimmung des Vorliegens oder Nichtvorliegens des mindestens einen SNPs durch Hybridisierung eines PCR-Produkts an eine allelspezifische Polynucleotidsonde unter stringenten Bedingungen erfolgt.

6. Verwendung einer Oligonucleotidsonde, die zwischen 5 und 50 Nucleotide aufweist, und die einen SNP in einem der Gene TCF4 oder LRP6 spezifisch detektiert, wobei der SNP ausgewählt ist aus rs3814570, rs10885394, rs10885395und rs2302685 in einem Verfahren nach einem der Ansprüche 1 bis 5.

7. Verwendung einer Oligonucleotidsonde nach Anspruch 6, **dadurch gekennzeichnet, dass** mit ihr die Position 101 einer Nucleinsäuresequenz detektiert werden kann, die ausgewählt ist aus der Gruppe bestehend aus SEQ-ID-Nr. 10, SEQ-ID Nr. 19, SEQ-ID-Nr. 20, oder SEQ-ID-Nr. 21.

8. Verwendung eines SNPs in mindestens einem Gen, das für ein mit dem WNT-Signalweg in Paneth-Zellen assoziiertes Proteins kodiert, zur Bestimmung einer Prädisposition eines Lebewesens für chronisch entzündliche Darmerkrankungen, wobei das SNP ausgewählt ist aus der Gruppe umfassend rs3814570, rs10885394, rs10885395 und rs2302685.

9. Verwendung nach Anspruch 8, **dadurch gekennzeichnet, dass** die entzündliche Darmerkrankung Morbus Crohn ist.

10. Verwendung nach einem der Ansprüche 8 oder 9, **dadurch gekennzeichnet, dass** ein SNP mit der Nummer rs2302685 verwendet wird, um die Prädisposition eines Patienten zur Entwicklung einer Crohn's Erkrankung des Ileums mit frühzeitigem Ausbruch zu bestimmen.

11. Verfahren nach einem der Ansprüche 1 bis 5 zur differentiellen Diagnose entzündlicher Darmerkrankungen zur Unterscheidung von Morbus Crohn und den jeweils anderen entzündlichen bzw. infektiösen Darmerkrankungen.

## Claims

1. Method for determining a predisposition of an organism for the development of Crohn's Disease, the method comprising determining, in a biological specimen of the organism, the presence of at least one single nucleotide polymorphism (SNP), **characterized in that** the at least one SNP is determined in at least one gene coding for a protein associated with the Wnt signaling pathway in Paneth cells, wherein the at least one gene is TCF4 or LRP6 and wherein the at least one SNP is selected from any of the following:
rs3814570, rs10885394, rs10885395 (TCF 4) and rs2302685 (LRP6).

2. Method according to Claim 1, **characterized in that** the predisposition of an organism for Crohn's Disease of the small intestine is determined.

3. Method according to either of Claims 1 and 2, in which the presence or absence of the at least one SNP is determined by primer extension of a PCR product and MALDI-TOF analysis.

4. Method according to either of Claims 1 and 2, in which the presence or absence of the at least one SNP is determined by sequencing of a PCR product.

5. Method according to either of Claims 1 and 2, in which the presence or absence of the at least one SNP is determined by hybridization of a PCR product to an allele-specific polynucleotide probe under stringent conditions.

6. Use of an oligonucleotide probe having between 5 and 50 nucleotides, which specifically detects an SNP in either of the genes TCF4 and LRP6, wherein the SNP is selected from rs3814570, rs10885394, rs10885395 and rs2302685, in a method according to any of Claims 1 to 5.

7. Use of an oligonucleotide probe according to Claim 6, **characterized in that** it is able to detect position 101 of a nucleic acid sequence selected from the group consisting of SEQ ID No. 10, SEQ ID No. 19, SEQ ID No. 20 or SEQ ID No. 21.

8. Use of an SNP in at least one gene coding for a protein associated with the Wnt signaling pathway in Paneth cells, for determining a predisposition of an organism for chronic inflammatory bowel diseases, wherein the SNP is selected from the group comprising rs3814570, rs10885394, rs 10885395 and rs2302685.

9. Use according to Claim 8, **characterized in that** the inflammatory bowel disease is Crohn's Disease.

10. Use according to either of Claims 8 and 9, **characterized in that** an SNP having the number rs2302685 is used in order to determine the predisposition of a patient for developing early onset Crohn's disease of the ileum.

11. Method according to any of Claims 1 to 5 for differential diagnosis of inflammatory bowel diseases for distinguishing Crohn's Disease and the respective other inflammatory or infectious bowel diseases.

## Revendications

1. Procédé de détermination d'une prédisposition d'un être vivant au développement de la maladie de Crohn, selon lequel la présence d'au moins un polymorphisme d'un seul nucléotide (SNP) est déterminée dans un échantillon biologique de l'être vivant, **caractérisé en ce que** ledit au moins un SNP est déterminé dans au moins un gène qui code pour une protéine associée avec la voie de signalisation Wnt dans les cellules de Paneth, ledit au moins un gène étant TCF4 ou LRP6, et ledit au moins un SNP étant choisi parmi les suivants : rs3814570, rs10885394, rs10885395 (TCF 4) et rs2302685 (LRP6).

2. Procédé selon la revendication 1, **caractérisé en ce que** la prédisposition d'un être vivant à la maladie de Crohn de l'intestin grêle est déterminée.

3. Procédé selon l'une quelconque des revendications 1 ou 2, dans lequel la détermination de la présence ou de la non-présence dudit au moins un SNP a lieu par allongement d'amorce d'un produit de PCR et analyse MALDI-TOF.

4. Procédé selon l'une quelconque des revendications 1 ou 2, dans lequel la détermination de la présence ou de la non-présence dudit au moins un SNP a lieu par séquençage d'un produit de PCR.

5. Procédé selon l'une quelconque des revendications 1 ou 2, dans lequel la détermination de la présence ou de la non-présence dudit au moins un SNP a lieu par hybridation d'un produit de PCR sur une sonde polynucléotidique spécifique d'allèle dans des conditions stringentes.

6. Utilisation d'une sonde oligonucléotidique, qui comprend entre 5 et 50 nucléotides, et qui détecte spécifiquement un SNP dans un des gènes TCF4 ou LRP6, le SNP étant choisi parmi rs3814570, rs10885394, rs10885395 et rs2302685, par un procédé selon l'une quelconque des revendications 1 à 5.

7. Utilisation d'une sonde oligonucléotidique selon la revendication 6, **caractérisée en ce qu'**elle permet de détecter la position 101 d'une séquence d'acides nucléiques, choisie dans le groupe constitué par SEQ ID NO : 10, SEQ ID NO : 19, SEQ ID NO : 20 ou SEQ ID NO : 21.

8. Utilisation d'un SNP dans au moins un gène, qui code pour une protéine associée avec la voie de signalisation WNT dans des cellules de Paneth, pour la détermination d'une prédisposition d'un être vivant à des maladies intestinales inflammatoires chroniques, le SNP étant choisi dans le groupe comprenant rs3814570, rs10885394, rs10885395 et rs2302685.

9. Utilisation selon la revendication 8, **caractérisée en ce que** la maladie intestinale inflammatoire est la maladie de Crohn.

10. Utilisation selon l'une quelconque des revendications 8 ou 9, **caractérisée en ce qu'**un SNP de numéro rs2302685 est utilisé pour déterminer la prédisposition d'un patient à développer une maladie de Crohn de l'iléon avec apparition précoce.

11. Procédé selon l'une quelconque des revendications 1 à 5 pour le diagnostic différentiel de maladies intestinales inflammatoires afin de différencier la maladie de Crohn des autres maladies intestinales inflammatoires ou infectieuses.
